# EUROPEAN PATENT SPECIFICATION

(11) **EP 0 785 948 B1**
(45) Date of publication and mention of the grant of the patent: **16.04.2003**
(21) Application number: 95934793.1
(22) Date of filing: 12.10.1995
(51) Int. Cl.: C07K 14/50, C12N 15/12, A61K 38/18

(54) **ANALOGS OF KERATINOCYTE GROWTH FACTOR**
ANALOGEN DES KERATINOZYTENWACHSTUMFAKTORS
ANALOGUES DU FACTEUR DE CROISSANCE DE KERATINOCYTES

(30) Priority: 13.10.1994 US 323340; 13.10.1994 US 323475; 07.06.1995 US 487825
(43) Date of publication of application: 30.07.1997
(73) Proprietor: Amgen Inc.,, Thousand Oaks, California 91320-1799 (US)
(72) Inventor: MORRIS, Charles F., Newbury Park, CA 91320 (US); KENNEY, William C., Thousand Oaks, CA 91360 (US); CHEN, Bao-Lu, Emeryville, CA 94608 (US); HSU, Eric W., Thousand Oaks, CA 91320 (US)
(74) Representative: Grünecker, Kinkeldey, Stockmair & Schwanhäusser Anwaltssozietät
(86) International application number: IB9500971
(87) International publication number: WO96011949

(56) References cited:
- EP-A- 0 510 662
- WO-A-90/08771
- WO-A-95/01434
- WO-A-95/03831
- US-A- 4 959 314
- JOURNAL OF BIOLOGICAL CHEMISTRY, vol. 268, no. 4, 5 February 1993, MD US, pages 2984-2988, XP002008659 D.RON ET AL: "Expression of biologically active rKGF" cited in the application
- BIOCHEMICAL AND BIOPHYSICAL RESEARCH COMMUNICATIONS, vol. 205, no. 1, 30 November 1994, ORLANDO, FL US, pages 872-879, XP002008660 L.A.BARE ET AL: "Effect of cysteine substitutions on the mitogenic activity and stability of rKGF"
- BIOCHEMICAL AND BIOPHYSICAL RESEARCH COMMUNICATIONS, vol. 151, no. 2, 15 March 1988, ORLANDO, FL US, pages 701-708, XP002008661 N.SENO T AL: "Stabilizing bFGF using protein engineering"

## Description

### Field of the Invention

The present invention relates to recombinant DNA technology and protein engineering. Specifically, recombinant DNA methodologies have been applied to generate polypeptide analogs of keratinocyte growth factor (KGF), a potent mitogen of non-fibroblast epithelial cell growth, wherein the analogs have improved stability as compared to that of the parent KGF.

### Background of the Invention

The complex process of tissue generation and regeneration is mediated by a number of protein factors sometimes referred to as soft tissue growth factors. These molecules are generally released by one cell type and act to influence proliferation of other cell types. (Rubin et al. (1989), *Proc. Nat'l. Acad. Sci. USA,* 86:802-806). Some soft tissue growth factors are secreted by particular cell types and influence the proliferation, differentiation, and/or maturation of responsive cells in the development of multicellular organisms (Finch *et al.* (1989), *Science,* 245:752-755). In addition to their roles in developing organisms, some are significant in the continued health and maintenance of more mature systems. For instance, in mammals, there are many systems where rapid cell turnover occurs. Such systems include the skin and the gastrointestinal tract, both of which are comprised of epithelial cells. Included within this group of soft tissue growth factors is a protein family of fibroblast growth factors (FGFs).

There are currently eight known FGF family members which share a relatedness among primary structures: basic fibroblast growth factor, bFGF (Abraham *et al.* (1986), *EMBO J*., 5:2523-2528); acidic fibroblast growth factor, aFGF (Jaye *et al.* (1986), *Science,* 233:541-545); int-2 gene product, int-2 (Dickson & Peters (1987), *Nature,* 326:833); hst/kFGF (Delli-Bovi et *al.* (1987), *Cell,* 50:729-737) and Yoshida *et al.* (1987), *Proc. Natl. Acad. Sci. USA,* 84:7305-7309); FGF-5 (Zhan *et al.* (1988), *Mol. Cell. Biol.,* 8:3487-3495); FGF-6 (Marics *et al.* (1989), *Oncogene,* 4:335-340); keratinocyte growth factor (Finch *et al*. (1989), *Science,* 24:752-755); and hisactophilin (Habazzettl *et al*. (1992), *Nature,* 359:855-858).

Among the FGF family of proteins, keratinocyte growth factor ("KGF") is a unique effector of non-fibroblast epithelial (particularly keratinocyte) cell proliferation derived from mesenchymal tissues. The term "native KGF" refers to a natural human (hKGF) or recombinant (rKGF) polypeptide (with or without a signal sequence) as depicted by the amino acid sequence presented in SEQ ID NO:2 or an allelic variant thereof. [Unless otherwise indicated, amino acid numbering for molecules described herein shall correspond to that presented for the mature form of the native molecule (i.e., minus the signal sequence), as depicted by amino acids 32 to 194 of SEQ ID NO:2.]

Native KGF may be isolated from natural human sources (hKGF) or produced by recombinant DNA techniques (rKGF) (Finch et *al*. (1989), *supra;* Rubin *et al*. (1989), *supra;* Ron *et al*. (1993), *The Journal of Biological Chemistry,* 268(4):2984-2988; and Yan *et al*. (1991), *In Vitro Cell. Dev. Biol.,* 27A:437-438).

It is known that native KGF is relatively unstable in the aqueous state and that it undergoes chemical and physical degradation resulting in a loss of biological activity during processing and storage (Chen *et al.* (1994), *Pharmaceutical Research,* 11:1582-1589). Native KGF is prone also to aggregation at elevated temperatures and it becomes inactivated under acidic conditions (Rubin *et al*. (1989), *Proc. Natl. Acad. Sci. USA,* 86:802-806). Aggregation of native KGF in aqueous solution also results in inactivated protein. This is disadvantageous because such loss of activity makes it impractical to store aqueous formulations of native KGF proteins for extended periods of time or to administer the protein over extended periods. Moreover, this is particularly problematic when preparing pharmaceutical formulations, because aggregated proteins have been known to be immunogenic (Cleland *et al.* (1993), *Crit. Rev. Therapeutic Drug Carrier Systems,* 10:307-377; Robbins *et al.* (1987), *Diabetes,* 36:838-845; and Pinckard *et al.* (1967), *Clin. Exp. Immunol.,* 2:331-340).

Native KGF comprises five cysteine residues, namely amino acids 1, 15, 40, 102, and 106 (Finch *et al.* (1989), *Science,* 24:752-755). Although the cysteine content of native KGF was reported, what has not been reported are the roles played by cysteine residues with respect to activity (e.g., essentiality to biological activity) and tertiary structure (e.g., proclivity to form undesirable intermolecular or intramolecular disulfide bonds). Thus, there is no prior art teaching of which, if any, cysteine residues are essential or are involved in undesirable disulfide bond formation, making the protein susceptible to aggregation and/or instability.

In order to attempt to improve or otherwise alter one or more of the characteristics of native KGF, protein engineering may be employed. Recombinant DNA technology has been utilized to modify the sequences of native KGF.

Ron *et al.* (1993), *J. Biol. Chem.,* 268(4):2984-2988 reported modified KGF polypeptides having 3, 8, 27, 38 or 49 amino acids deleted from the N-terminus. Those polypeptides missing 3, 8, or 27 N-terminal residues retained heparin binding ability; the others did not. Also, the polypeptides missing 3 and 8 residues were reported as being fully active, whereas the form missing 27 residues was 10-20 fold less mitogenic, and the forms lacking 38 or 49 amino acids did not have mitogenic activity. The stability of the modified KGF polypeptides was not discussed or otherwise reported.

Published PCT application no. 90/08771, *supra,* also reported the production of a chimeric protein wherein about the first 40 N-terminal amino acids of the mature form of native KGF were combined with the C-terminal portion (about 140 amino acids) of aFGF. The chimera was reported to target keratinocytes like KGF, but it lacked susceptibility to heparin, a characteristic of aFGF but not KGF. The stability of the chimera was not discussed or otherwise reported. WO95/01434 teaches a truncated form of KGF lacking 23 N-terminal amino acids, its use in stimulating cells, its DNA and recombinant production thereof.

The literature has not reported a modified KGF molecule having significantly improved stability relative to native KGF. Moreover, the literature has not reported sufficient teachings or evidence to provide a reasonable expectation of successfully generating KGF molecules with such desirable characteristics.

Generally, the effects upon biological activity of any amino acid change upon a protein will vary depending upon a number of factors, including the three-dimensional structure of the protein and whether or not the modifications are to the receptor binding region of the primary sequence of the protein. As neither the three-dimensional structure nor the receptor binding region on the primary structure of native KGF has been published, the knowledge within the art does not permit generalization about the effects of amino acid modifications to native KGF based upon the effects of amino acid modifications on even commonly categorized proteins.

It is the object of this invention to provide polypeptide molecules encoding such analogs that exhibit enhanced stability (e.g., when subjected to typical pH, thermal and/or other storage conditions) as compared to native KGF.

### Summary of the Invention

The present invention provides a polypeptide analog of native keratinocyte growth factor termed "KGF," wherein native KGF corresponds to the following sequence 
Cys Asn Asp Met Thr Pro Glu Gln Met Ala Thr Asn Val Asn Cys Ser Ser
1               5                   10                  15

Pro Glu Arg His Thr Arg Ser Tyr Asp Tyr Met Glu Gly Gly Asp Ile
        20                  25                  30

Arg Val Arg Arg Leu Phe Cys Arg Thr Gln Trp Tyr Leu Arg Ile Asp
    35                  40                  45

Lys Arg Gly Lys Val Lys Gly Thr Gln Glu Met Lys Asn Asn Tyr Asn
50                  55                  60                  65

Ile Met Glu Ile Arg Thr Val Ala Val Gly Ile Val Ala Ile Lys Gly
                70                  75                  80

Val Glu Ser Glu Phe Tyr Leu Ala Met Asn Lys Glu Gly Lys Leu Tyr
            85                  90                  95

Ala Lys Lys Glu Cys Asn Glu Asp Cys Asn Phe Lys Glu Leu Ile Leu
        100                 105                 110

Glu Asn His Tyr Asn Thr Tyr Ala Ser Ala Lys Trp Thr His Asn Gly
    115                 120                 125

Gly Glu Met Phe Val Ala Leu Asn Gln Lys Gly Ile Pro Val Arg Gly
130                 135                 140                 145

Lys Lys Thy Lys Lys Glu Gln Lys Thr Ala His Phe Leu Pro Met Ala
150 155 160

Ile Thr,
 with or without a signal sequence, wherein the analog comprises the amino acid sequence of KGF comprising a deletion, substitution and/or insertion of one or more of amino acids 1-24 whereby the cysteine residue at amino acid position 1 and the cysteine residue at amino acid position 15 are each either deleted or substituted with another amino acid. optionally further having an N-terminal methionine residue and/or further comprising a charge-change substitution at an amino acid position selected from Arg (41), Glu (43), Lys (55), Lys (95), Asn (137), Gln (138), Lys (139), Arg (144), Lys (147), Gln (152), Lys (153) or Thr (154), or in case of Δ23-KGF having a charge-change modification of His (116), or in case of native KGF having both Cys (1) and Cys (15) substituted by Ser optionally having also Cys (40) or Cys (102) or both Cys (102) and Cys (106) substituted by Ser with the proviso that the analog is not the keratinocyte growth factor protein consisting of amino acids 24-163.

Surprisingly, it has been discovered when a KGF molecule (i.e., parent molecule) having cysteine residues corresponding (as determined using techniques described above) to Cys¹ and Cys¹⁵ of native KGF (cysteine residues 32 and 46 of SEQ ID NO:2) is modified by replacing the corresponding cysteines, the resultant KGF analog has improved stability as compared to the parent molecule. Preferably, in addition to having increased stability, the invention is directed to those analogs which also exhibit full biological activity (i.e., at least substantially similar receptor binding or affinity) as compared to native KGF.

In another aspect of the invention, purified and isolated nucleic acid molecules encoding the various biologically active polypeptide analogs of KGF are described. In one embodiment, such nucleic acids comprise DNA molecules cloned into biologically functional plasmid or viral vectors. In another embodiment, nucleic acid constructs may then be utilized to stably transform a procaryotic or eucaryotic host cell. In still another embodiment, the invention involves a process wherein either a procaryotic (preferably *E. coli*) or eucaryotic host cell stably transformed with a nucleic acid molecule is grown under suitable nutrient conditions in a manner allowing the expression of the KGF analog. Following expression, the resultant recombinant polypeptide can be isolated and purified.

A further aspect of the invention concerns pharmaceutical formulations comprising a therapeutically effective amount of a KGF analog and an acceptable pharmaceutical carrier. Such formulations will be useful in treating patients afflicted with epithelial diseases and injuries.

In this vein, another aspect relates to the use of a KGF analog for preparing a medicament for the treatment of disease requiring the stimulation of the production of non-fibroblast epithelial cells. Such epithelial cells include various adnexal cells, pancreatic cells, liver cells, and mucosal epithelium in the respiratory and gastrointestinal tracts.

### Brief Description of the Figures

When making references to Fig. 4, 7-24 and 37-50 and the specific amino acid position, the initial methionine in the sequence should be considerd residue number "O".

Figure 1 shows the nucleotide (SEQ ID NO:1) and amino acid (SEQ ID NO:2) sequences of native KGF (the nucleotides encoding the mature form of native KGF is depicted by bases 201 to 684 of SEQ ID NO:1 and the mature form of KGF is depicted by amino acid residues 32 to 194 of SEQ ID NO:2).

Figures 2A, 2B and 2C show the plasmid maps of pCFM1156, pCFM1656 and pCFM3102, respectively.

Figure 3 shows the nucleotide (SEQ ID NO:3) and amino acid (SEQ ID NO:4) sequences of the construct RSH-KGF.

Figure 4 shows the nucleotide (SEQ ID NO:5) and amino acid (SEQ ID NO:6) sequences of the construct contained in plasmid KGF.

Figure 5 shows the chemically synthesized OLIGOs (OLIGO#6 through OLIGO#11; SEQ ID NO:12-17, respectively) used to substitute the DNA sequence between a *KpnI* site and an *EcoR*I site (from amino acid positions 46 to 85 of SEQ ID No:6) in the construct contained in plasmid KGF to produce the construct in plasmid KGF(dsd).

Figure 6 shows the chemically synthesized OLIGOs (OLIGO#12 through OLIGO#24; SEQ ID NO:18-30, respectively) used to construct KGF (codon optimized).

Figure 7 shows the nucleotide (SEQ ID NO:31) and amino acid (SEQ ID NO:32) sequences of C(1,15)S, a KGF analog having substitutions of serine for cysteine at amino acid positions 1 and 15 of native KGF.

Figure 8 shows the nucleotide (SEQ ID NO:33) and amino acid (SEQ ID NO:34) sequences of ΔN3/C(15)S, a KGF analog having a deletion of the first 3 amino acids of the N-terminus and a substitution of serine for cysteine at amino acid position 15 of native KGF.

Figure 9 shows the nucleotide (SEQ ID NO:35) and amino acid (SEQ ID NO:36) sequences of ΔN3/C(15)-, a KGF analog having a deletion of the first 3 amino acids of the N-terminus and a deletion of cysteine at amino acid position 15 of native KGF.

Figure 10 shows the nucleotide (SEQ ID NO:37) and amino acid (SEQ ID NO:38) sequences of ΔN8/C(15)S, a KGF analog having a deletion of the first 8 amino acids of the N-terminus and a substitution of serine for cysteine at amino acid position 15 of native KGF.

Figure 11 shows the nucleotide (SEQ ID NO:39) and amino acid (SEQ ID NO:40) sequences of ΔN8/C(15)-, a KGF analog having a deletion of the first 8 amino acids of the N-terminus and a deletion of cysteine at amino acid position 15 of native KGF.

Figure 12 shows the nucleotide (SEQ ID NO:41) and amino acid (SEQ ID NO:42) sequences of ΔN15, a KGF analog having a deletion of the first 15 amino acids of the N-terminus of native KGF.

Figure 13 shows the nucleotide (SEQ ID NO:43) and amino acid (SEQ ID NO:44) sequences of ΔN16, a KGF analog having a deletion of the first 16 amino acids of the N-terminus of native KGF.

Figure 14 shows the nucleotide (SEQ ID NO:45) and amino acid (SEQ ID NO:46) sequences of ΔN17, a KGF analog having a deletion of the first 17 amino acids of the N-terminus of native KGF.

Figure 15 shows the nucleotide (SEQ ID NO:47) and amino acid (SEQ ID NO:48) sequences of ΔN18, a KGF analog having a deletion of the first 18 amino acids of the N-terminus of native KGF.

Figure 16 shows the nucleotide (SEQ ID NO:49) and amino acid (SEQ ID NO:50) sequences of ΔN19, a KGF analog having a deletion of the first 19 amino acids of the N-terminus of native KGF.

Figure 17 shows the nucleotide (SEQ ID NO:51) and amino acid (SEQ ID NO:52) sequences of ΔN20, a KGF analog having a deletion of the first 20 amino acids of the N-terminus of native KGF.

Figure 18 shows the nucleotide (SEQ ID NO:53) and amino acid (SEQ ID NO:54) sequences of ΔN21, a KGF analog having a deletion of the first 21 amino acids of the N-terminus of native KGF.

Figure 19 shows the nucleotide (SEQ ID NO:55) and amino acid (SEQ ID NO:56) sequences of ΔN22, a KGF analog having a deletion of the first 22 amino acids of the N-terminus of native KGF.

Figure 20 shows the nucleotide (SEQ ID NO:57) and amino acid (SEQ ID NO:58) sequences of ΔN23, a KGF analog having a deletion of the first 23 amino acids of the N-terminus of native KGF.

Figure 21 shows the nucleotide (SEQ ID NO:59) and amino acid (SEQ ID NO:60) sequences of ΔN24, a KGF analog having a deletion of the first 24 amino acids of the N-terminus of native KGF.

Figure 22 shows the nucleotide (SEQ ID NO:61) and amino acid sequences (SEQ ID NO:62) of C(1,15)S/R(144)E, a KGF analog having substitutions of serine for cysteine at amino acid positions 1 and 15 and a substitution of glutamic acid for arginine at amino acid position 144 of native KGF.

Figure 23 shows the nucleotide (SEQ ID NO:63) and amino acid (SEQ ID NO:64) sequences of C(1,15)S/R(144)Q, a KGF analog having substitutions of serine for cysteine at amino acid positions 1 and 15 and a substitution of glutamine for arginine at amino acid position 144 of native KGF.

Figure 24 shows the nucleotide (SEQ ID NO:65) and amino acid (SEQ ID NO:66) sequences of ΔN23/R(144)Q, a KGF analog having a deletion of the first 23 amino acids of the N-terminus and a substitution of glutamine for arginine at amino acid position 144 of native KGF.

Figure 25 shows the amount of remaining soluble protein when native KGF and C(1,15)S were stored in 20 mM NaPO₄, 0.15 M NaCl, pH 7.0 at 37°C for 27 hrs.

Figure 26 shows the amount of remaining soluble protein when native KGF and C(1,15)S were stored in 0.15 M NaCl, 20 mM NaPO₄, 0.15 M NaCl, pH 7.0 at 37°C for 27 hrs.

Figure 27 shows the amount of remaining soluble protein when native KGF and C(1,15)S were stored in 50 mM NaPO₄, 0.15 M NaCl, pH 7.0 at 37°C for 27 hrs.

Figure 28 shows the amount of soluble protein, of native KGF, C(1,15)S, C(1,15)S/R(144)E and C(1,15)S/R(144)Q determined by size exclusion HPLC, as a function of incubation time at 37°C.

Figure 29 shows the estimated melting temperature (Tₘ) as a function of pH for native KGF, C(1,15)S, C(1,15)S/R(144)Q and C(1,15)S/R(144)E.

Figure 30 shows a typical profile of mitogenic activity of C(1,15)S determined by measuring the incorporation of [³H]-Thymidine during DNA synthesis by comparing it to a native KGF standard curve.

Figure 31 shows a typical profile of the mitogenic activity of ΔN15 determined by measuring the incorporation of [³H]-Thymidine during DNA synthesis and by comparing it to a native KGF standard curve.

Figure 32 shows a typical profile of the mitogenic activity of ΔN23 determined by measuring the incorporation of [³H]-Thymidine during DNA synthesis and by comparing it to a native KGF standard curve.

Figure 33 shows a typical profile of the mitogenic activity of ΔN23/R(144)Q determined by measuring the incorporation of [³H]-Thymidine during DNA synthesis and by comparing it to a native KGF standard curve.

Figure 34 shows a typical profile of the mitogenic activity of C(1,15)S/R(144)Q determined by measuring the incorporation of [³H]-Thymidine during DNA synthesis and by comparing it to a native KGF standard curve.

Figure 35 shows a typical profile of the mitogenic activity of C(1,15)S/R(144)E determined by measuring the incorporation of [³H]-Thymidine during DNA synthesis and by comparing it to a native KGF standard curve.

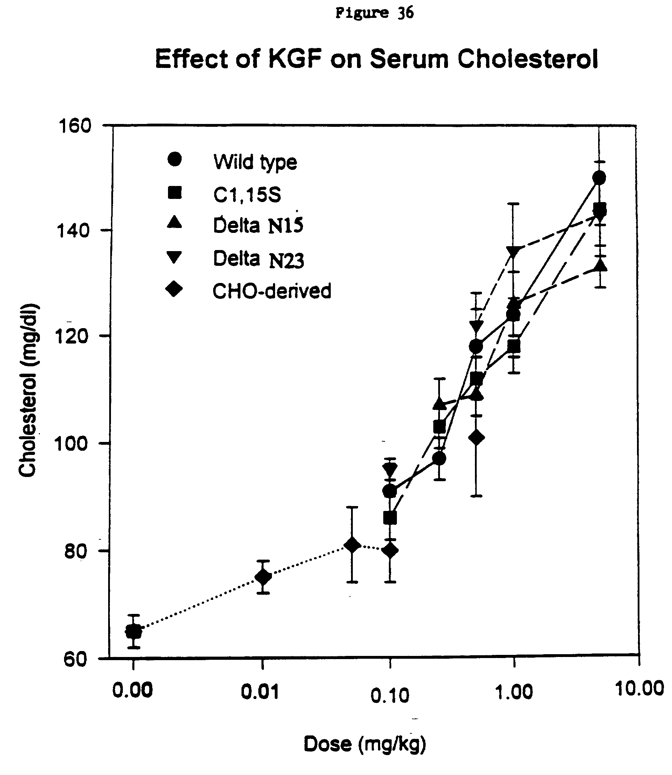

Figure 36 shows the effects of native (wild type) KGF, ΔN15, CHO-derived, C(1,15)S and ΔN23 on serum chemistries.

Figure 37 shows the nucleotide (SEQ ID NO:77) and amino acid (SEQ ID NO:78) sequences of C(1,15,40)S, a KGF analog having substitutions of serine for cysteine at amino acid positions 1, 15 and 40 of native KGF.

Figure 38 shows the nucleotide (SEQ ID NO:79) and amino acid (SEQ ID NO:80) sequences of C(1,15,102)S, a KGF analog having substitutions of serine for cysteine at amino acid positions 1, 15 and 102 of native KGF.

Figure 39 shows the nucleotide (SEQ ID NO:81) and amino acid (SEQ ID NO:82) sequences of C(1,15,102,106)S, a KGF analog having substitutions of serine for cysteine at amino acid positions 1, 15, 102 and 106 of native KGF.

Figure 40 shows the nucleotide (SEQ ID NO:83) and amino acid (SEQ ID NO:84) sequences of ΔN23/N(137)E, a KGF analog having a deletion of the first 23 amino acids of the N-terminus and a substitution of glutamic acid for asparagine at amino acid position 137 of native KGF.

Figure 41 shows the nucleotide (SEQ ID NO:85) and amino acid (SEQ ID NO:86) sequences of ΔN23/K(139)E, a KGF analog having a deletion of the first 23 amino acids of the N-terminus and a substitution of glutamic acid for lysine at amino acid position 139 of native KGF.

Figure 42 shows the nucleotide (SEQ ID NO:87) and amino acid (SEQ ID NO:88) sequences of ΔN23/K(139)Q, a KGF analog having a deletion of the first 23 amino acids of the N-terminus and a substitution of glutamine for lysine at amino acid position 139 of native KGF.

Figure 43 shows the nucleotide (SEQ ID NO:89) and amino acid (SEQ ID NO:90) sequences of ΔN23/R(144)A, a KGF analog having a deletion of the first 23 amino acids of the N-terminus and a substitution of alanine for arginine at amino acid position 144 of native KGF.

Figure 44 shows the nucleotide (SEQ ID NO:91) and amino acid (SEQ ID NO:92) sequences of ΔN23/R(144)E, a KGF analog having a deletion of the first 23 amino acids of the N-terminus and a substitution of glutamic acid for arginine at amino acid position 144 of native KGF.

Figure 45 shows the nucleotide (SEQ ID NO:93) and amino acid (SEQ ID NO:94) sequences of ΔN23/R(144)L, a KGF analog having a deletion of the first 23 amino acids of the N-terminus and a substitution of leucine for arginine at amino acid position 144 of native KGF.

Figure 46 shows the nucleotide (SEQ ID NO:95) and amino acid (SEQ ID NO:96 sequences of ΔN23/K(147)E, a KGF analog having a deletion of the first 23 amino acids of the N-terminus and a substitution of glutamic acid for lysine at amino acid position 147 of native KGF.

Figure 47 shows the nucleotide (SEQ ID NO:97) and amino acid (SEQ ID NO:98) sequences of ΔN23/K(147)Q, a KGF analog having a deletion of the first 23 amino acids of the N-terminus and a substitution of glutamine for lysine at amino acid position 147 of native KGF.

Figure 48 shows the nucleotide (SEQ ID NO:99) and amino acid (SEQ ID NO:100) sequences of ΔN23/K(153)E, a KGF analog having a deletion of the first 23 amino acids of the N-terminus and a substitution of glutamic acid for lysine at amino acid position 153 of native KGF.

Figure 49 shows the nucleotide (SEQ ID NO:101) and amino acid (SEQ ID NO:102) sequences of ΔN23/K(153)Q, a KGF analog having a deletion of the first 23 amino acids of the N-terminus and a substitution of glutamine for lysine at amino acid position 153 of native KGF.

Figure 50 shows the nucleotide (SEQ ID NO:103) and amino acid (SEQ ID NO:104) sequences of ΔN23/Q(152)E/K(153)E, a KGF analog having a deletion of the first 23 amino acids of the N-terminus and a substitution of glutamic acid for glutamine at amino acid position 152 of native KGF and glutamic acid for lysine at amino acid position 153 of native KGF.

Figure 51 shows the effect of ΔN23 on streptozotocin-induced diabetes in Sprague-Dawley rats.

### Detailed Description

In accordance with the present invention, novel analogs of KGF are provided. It has now been determined that four of the cysteine residues of native KGF (Cys¹ and Cys¹⁵, and Cys¹⁰² and Cys¹⁰⁶⁾ are involved in the formation of two disulfide bridges. Cys⁴⁰ is not involved in intramolecular disulfide formation. Hence, KGF contains two small disulfide loops separated by almost 90 amino acids. Based upon first principals, the determination of which cysteine residues are involved in the disulfide bridges suggests which cysteines are free to form undesirable intermolecular crosslinks or intramolecular bonds that cause the protein to adopt an undesirable tertiary structure (e.g., a conformation that reduces the activity of the protein).

Surprisingly, it has been found that modifying a KGF by deleting or substituting amino acid residues for the cysteine residues corresponding to positions 1 and 15 of KGF (positions 32 and 46 of SEQ ID NO:2) produces a KGF analog having substantially improved stability (i.e., reduced problems caused by improper refolding, intermolecular disulfide formation and/or protein aggregation). For example, the KGF analogs will generally be purified in a greater yield of soluble, correctly folded protein. Moreover, once the material is purified, it will be more stable to pH, temperature, etc. as compared to the stability of the parent molecule. Although not intended to be bound by theory, it is believed that Cys¹ and Cys¹⁵ of KGF in addition to forming an intramolecular disulfide bridge and an N-terminal disulfide loop, under certain conditions also exist as free cysteines which are capable of forming intermolecular disulfide bridges, resulting in protein instability and aggregation. Moreover, it has been discovered that deletion of the N-terminal disulfide loop is not important for receptor binding or mitogenic activity.

As used in this invention, a "KGF analog" or a "polypeptide analog of KGF" shall mean any of the described naturally and non-naturally occurring polypeptides differing in structure from a KGF by possessing modifications in peptide sequence corresponding to the 24 amino acid N-terminus of the KGF , wherein Cys¹ and Cys¹⁵ of the KGF are replaced or deleted. The manner by which the cysteines are replaced or deleted is not significant and includes, for example, polypeptide analogs incorporating one or more amino acid deletions and/or substitutions. Accordingly, the invention provides a family of novel keratinocyte growth factor proteins. This family comprises several groups of proteins.

One group of KGF analogs includes molecules in which the cysteines at positions 1 and 15 of a KGF are replaced with amino acids, including those that do not occur naturally in proteins. Strategies for generating the substitution analogs include using site-directed mutagenesis (Ho *et al.* (1989), *Gene,* 77:51-59; Innis *et al*. "PRC Protocols", Academic Press, Inc., San Diego, CA). [KGF analogs comprising an amino acid substitution are referred to by the residue found at that position in the mature protein (minus signal sequence) set forth in SEQ ID NO:2, followed by that amino acid position in parentheses and the new amino acid. For instance, an analog comprising a cysteine to serine substitution at amino acid position 15 of KGF is referred to as "C(15)S".] Preferably, the cysteine is converted to a neutral amino acid such as glycine, valine, alanine, leucine, isoleucine, tyrosine, phenylalanine, histidine, tryptophan, serine, threonine and methionine, with serine, threonine and alanine being most preferred because of their chemical similarity to cysteine. Example 1 details the generation of C(1,15)S using partial gene synthesis (in conjunction with other recombinant techniques) followed by recombinant expression in a stably transformed bacterial host.

Another group of KGF analogs includes molecules that have the cysteines at positions 1 and 15 deleted from KGF. Different strategies may be employed in developing such KGF analogs, such as N-terminal truncations and site-specific deletions, or a combination of both.

An "N-terminal truncation" refers to a modification of a KGF wherein 1 to 24 N-terminal amino acid residues of the KGF , including Cys¹ and Cys¹⁵, have been deleted. [KGF analogs comprising a truncation of amino acids will be referred to by the residue deleted at that position in the mature protein (minus signal sequence) set forth in SEQ ID NO:2, beginning with the site of the deletion and by the number of residues deleted. For instance, a KGF analog comprising an N-terminal truncation of 24 residues of the KGF will be referred to as a "ΔN24" analog.] Specifically included within this group are ΔN23 analogs of native KGF wherein one or more of amino acid residues 41-154 are substituted with a neutral residue or negatively charged residue selected to effect a protein with a reduced positive charge, selected from Arg⁴¹, Gln⁴³, Lys⁵⁵, Lys⁹⁵, Lys¹²⁸, Asn¹³⁷, Gln¹³⁸, Lys¹³⁹, Arg¹⁴⁴, Lys¹⁴⁷, Gln¹⁵², Lys¹⁵³ or Thr¹⁵⁴, with Gln¹³⁸, Lys¹³⁹, Arg¹⁴⁴, Lys¹⁴⁷, Gln¹⁵² or Lys¹⁵³ and Arg¹⁴⁴ being most preferred Also included within this group are ΔN23 analogs of native KGF having a charge-change modification of amino acid residue 116, more preferably the substitution of Gly at position 116.

Example 1 details the generation of systematic N-terminal truncations accomplished using partial gene synthesis, in conjunction with other recombinant techniques, followed by recombinant expression in a stably transformed bacterial host. Such exemplified N-terminal truncations include ΔN15 to ΔN24. Moreover, Example 3 details expression in mammalian cell culture cells of DNA encoding native KGF and heterogeneous N-terminal glycosylated isoforms, the purification of preferably a glycosylated isoform having an N-terminal truncation of amino acids 1-23 of the mature form of native KGF.

In contrast, a site-specific deletion refers to a modification of a KGF wherein one or more amino acid residues (e.g., Cys¹ or Cys¹⁵) are removed. When a Cys¹ or Cys¹⁵ of the KGF is specifically deleted, the analog is one amino acid shorter than the KGF. [KGF analogs comprising an amino acid deletion are referred to by the residue found at that position in the mature protein (minus signal sequence) , followed by that amino acid position in parentheses, and a negative sign. For instance, an analog within this group comprising a deletion of cysteine at position 15 of the KGF is referred to as "C(15)-".] The site-specific deletions may be generated using site directed mutagenesis, as described above.

Also included within this group are analogs in which Cys¹ and Cys¹⁵ of a KGF are removed through truncation and deletion. For example, representative KGF analogs comprise the truncation of the first three amino terminal residues (Cys-Asn-Asp) of the KGF or the truncation of the first eight amino acids (Cys-Asn-Asp-Met-Thr-Pro-Glu-Gln) of the KGF coupled with the deletion of cysteine at amino acid position 15 of KGF (these analogs are referred to as ΔN3/C(15)- and ΔN8/C(15)-, respectively). As a methionine residue occurs naturally at the fourth and ninth amino acid position in native KGF, no additional Met codon is required to enable proper initiation of translation.

A still further group includes molecules in which the cysteines at positions 1 and 15 of KGF are replaced through truncation and substitution. For example, representative KGF analogs are ΔN3/C(15)S, and ΔN8/C(15)S. Such analogs comprise the truncation of the first three amino-terminal residues of a KGF or the deletion of the first eight amino-terminal residues of a KGF coupled with the substitution of cysteine at amino acid position 15 with another amino acid, for instance, serine.

When the KGF analogs are biologically generated, i.e., are the products of cellular expression as opposed to the products of solid state synthesis, proteolytic or enzymatic derivatization of naturally-occurring products, etc., the nucleic acids encoding such polypeptides, will differ in one or more nucleotides as compared to the native KGF nucleotide sequence. Such poly-nucleotides may be expressed and the resultant polypeptide purified by any one of a number of recombinant technology methods known to those skilled in the art.

DNA sequences coding for all or part of the KGF analogs may include among other things the incorporation of codons "preferred" for expression in selected host cells (e.g., "*E. coli* expression codons"); the provision of sites for cleavage by restriction enzymes; and the provision of additional initial, terminal, or intermediate nucleotide sequences (e.g., an initial methionine amino acid residue for expression in *E. coli* cells), to facilitate construction of readily expressed vectors.

The present invention also provides recombinant molecules or vectors for use in the method of expression of the polypeptides. Such vectors may be comprised of DNA or RNA and can be circular, linear, single-stranded or double-stranded in nature and can be naturally-occurring or assemblages of a variety of components, be they naturally-occurring or synthetic.

Many examples of such expression vectors are known. The components of the vectors, e.g. replicons, selection genes, enhancers, promoters, and the like, may be obtained from natural sources or synthesized by known procedures. In each case, expression vectors useful in this invention will contain at least one expression control element functionally associated with the inserted nucleic acid molecule encoding the KGF polypeptide analog. This control element is responsible for regulating polypeptide expression from the nucleic acid molecules of the invention. Useful control elements include, for example, the lac system, the trp system, the operators and promoters from phage λ, a glycolytic yeast promoter, a promoter from the yeast acid phosphatase gene, a yeast alpha-mating factor, and promoters derived from adenovirus, Epstein-Barr virus, polyoma, and simian virus as well as those from various retroviruses. However, numerous other vectors and control elements suitable for procaryotic or eucaryotic expression are known in the art and may be employed in the practice of this invention.

Examples of suitable procaryotic cloning vectors may include plasmids from *E. coli* (e.g. pBR322, col E1, pUC, and the F-factor), with preferred plasmids being pCFM1156 (ATCC 69702), pCFM1656 (ATCC 69576) and pCFM3102 (described in the Examples section, below). Other appropriate expression vectors of which numerous types are known in the art for mammalian, insect, yeast, fungal and bacterial expression can also be used for this purpose. The transfection of these vectors into appropriate host cells can result in expression of the KGF analog polypeptides.

Host microorganisms useful in this invention may be either procaryotic or eucaryotic. Suitable procaryotic hosts include various *E. coli* (e.g., FM5, HB101, DH5α, DH10, and MC1061), *Pseudomonas, Bacillus,* and *Streptomyces* strains, with *E. coli* being preferred. Suitable eucaryotic host cells include yeast and other fungi, insect cells, plant cells and animal cells, such as COS (e.g., COS-1 and COS-7) and CV-1 monkey cell lines, 3T3 lines derived from Swiss, Balb-c or NIH cells, HeLa and L-929 mouse cells, and CHO, BHK or HaK hamster cells. Depending upon the host employed, recombinant polypeptides produced in accordance herewith will be glycosylated with mammalian or other eucaryotic carbohydrates or may be non-glycosylated.

The preferred production method will vary depending upon many factors and considerations; the optimum production procedure for a given situation will be apparent to those skilled in the art through minimal experimentation. The resulting expression product may then be purified to near homogeneity using procedures known in the art. A typical purification procedure for procaryotic cell production involves rupturing the cell walls by high pressure or other means, centrifugation or filtration to remove cellular debris followed by ion exchange chromatography of supernatant or filtrate and, finally, hydrophobic interaction chromatography. If the analog is expressed in insoluble form, another purification technique involves first solublizing the inclusion bodies containing the analogs followed by ion exchange chromatography, then refolding of the protein, and, finally, hydrophobic interaction chromatography. Exemplary purification techniques are taught in commonly owned U.S.S.N. 08/323,339, filed on October 13, 1994. Generally, U.S.S.N. 08/323,339 teaches a method for purifying a keratinocyte growth factor comprising: (a) obtaining a solution comprising the KGF; (b) binding the KGF from the solution of part (a) to a cation exchange resin; (c) eluting the KGF in an eluate solution from the cation exchange resin; (d) either passing the eluate solution from part (c) through an appropriate molecular weight exclusion matrix or performing hydrophobic interaction chromatography on the eluate solution of part (c); and (e) recovering the KGF from the molecular weight exclusion matrix or hydrophobic interaction chromatography.

Of course, the analogs may be rapidly screened to assess their physical properties. The Examples section set forth various well-known stability assays, although the specific assay used to test the analog is not critical. Moreover, the level of biological activity (e.g., receptor binding and/or affinity, mitogenic, cell proliferative and/or *in vivo* activity) may also be tested using a variety of assays, some of which are set forth in the Examples section. Numerous assays are well-known and can be used to quickly screen the KGF analogs to determine whether or not they possess acceptable biological activity. One such assay specifically tests the KGF analogs for the ability to bind to the KGF receptor (KGFR) by competing with ¹²⁵I-KGF binding (Bottaro *et al.* (1990), *J. Biol. Chem.,* 265:12767-12770; Ron *et al.* (1993), *J. Biol. Chem.,* 268:2984-2988). An alternative method for assaying KGFR/KGF analog interactions involves the use of techniques such as real time biospecific interaction analysis (BIA) (Felder *et al.* (1993), *Molecular & Cellular Biology,* 13:1449-1455). Additionally a mitogenic assay can be utilized to test the ability of the KGF analogs to stimulate DNA synthesis (Rubin *et al.* (1989), *supra).* Finally, cell proliferative assays can be utilized to test the ability of the KGF analogs to stimulate cell proliferation (Falco, *et al*. (1988), *Oncogene,* 2:573-578). Using any of the aforementioned assay systems, KGF analogs can be rapidly screened for their biological activity.

In a preferred embodiment, the present invention is directed to KGF analogs which retain the full (i.e., at least substantially similar) *in vitro* or *in vivo* biological activity to that of native KGF. Exemplary KGF analogs with these properties, as determined by one or more of the above assays, are C(1,15)S, ΔN3/C(15)S, ΔN3/C(15)-, ΔN8/C(15)S, ΔN8/C(15)-, ΔN15, ΔN16, ΔN17, ΔN18, ΔN19, ΔN20, ΔN21, ΔN22, ΔN23, ΔN24 or ΔN23/R(144)Q.

The KGF analogs may be further modified to contain additional chemical moieties not normally a part of the peptide. Such derivatized moieties may improve the solubility, absorption, biological half life, and the like of the KGF analog. The moieties may alternatively eliminate or attenuate any undesirable side effects of the protein and the like. Moieties capable of mediating such effects are disclosed, for example, in *REMINGTON'S PHARMACEUTICAL SCIENCES,* 18th ed., Mack Publishing Co., Easton, PA (1990). Covalent modifications may be introduced into the molecule by reacting targeted amino acid residues of the peptide with an organic derivatizing agent that is capable of reacting with selected side chains or terminal residues (T.E. Creighton (1983), *PROTEINS: STRUCTURE AND MOLECULE PROPERTIES,* W.H. Freeman & Co., San Francisco, pp. 79-86). Polyethylene glycol ("PEG") is one such chemical moiety which has been used in the preparation of therapeutic protein products. For some proteins, the attachment of polyethylene glycol has been shown to protect against proteolysis, Sada, *et al.* (1991), J. Fermentation Bioengineering, 71:137-139 , and methods for attachment of certain polyethylene glycol moieties are available. See U.S. Patent No. 4,179,337, Davis et al., "Non-Immunogenic Polypeptides," issued December 18, 1979; and U.S. Patent No. 4,002,531, Royer, "Modifying enzymes with Polyethylene Glycol and Product Produced Thereby," issued January 11, 1977. For a review, see Abuchowski et al., in Enzymes as Drugs. (Holcerberg and Roberts, (eds.) pp. 367-383 (1981)). For polyethylene glycol, a variety of means have been used to attach the polyethylene glycol molecules to the protein. Generally, polyethylene glycol molecules are connected to the protein via a reactive group found on the protein. Amino groups, such as those on lysine residues or at the N-terminus, are convenient for such attachment. For example, Royer (U.S. Pat. No. 4,002,531, above) states that reductive alkylation was used for attachment of polyethylene glycol molecules to an enzyme. EP 0 539 167, published April 28, 1993, Wright, "Peg Imidates and Protein Derivates Thereof" states that peptides and organic compounds with free amino group(s) are modified with an imidate derivative of PEG or related water-soluble organic polymers. U.S. Patent No. 4,904,584, Shaw, issued February 27, 1990, relates to the modification of the number of lysine residues in proteins for the attachment of polyethylene glycol molecules via reactive amine groups.

In yet another embodiment, the present invention is directed to a single-dose administration unit of a medicinal formulation, which can be safely administered parenterally or orally to treat a disease in a warm-blooded animal (such as a human). Such medicinal formulation may be in the form of a lyophilized or otherwise dehydrated therapeutic or diagnostic which can be reconstituted by the addition of a physiologically acceptable solvent. The solvent may be any media such as sterile water, physiological saline solution, glucose solution or other aqueous carbohydrates (e.g., polyols such as mannitol, xylitol or glycerol) which is capable of dissolving the dried composition, which is compatible with the selected administration route and which does not negatively interfere with the active principle and the reconstitution stabilizers employed. In a specific embodiment, the present invention is directed to a kit for producing the single-dose administration unit. The kit contains both a first container having a dried protein and a second container having an aqueous formulation comprising a reconstitution stabilizer. As for the concentration of the protein in the solution, the solution volume which is charged into each container, and the capacity of the containers (interrelated parameters which can be suitably modified, depending upon the desired concentration of active principle in the end-dosage unit), these may vary within wide ranges well-known to skilled artisans.

KGF analogs according to the invention may be useful as therapeutic and diagnostic agents and as research reagents. Thus the KGF analogs may be used in *in vitro* and/or *in vivo* diagnostic assays to quantify the amount of KGF in a tissue or organ sample or to determine and/or isolate cells which express KGFR (Bottaro *et al*. (1990), *J. Biol. Chem*., 265:12767-12770; Ron *et al*. (1993), *J. Biol. Chem.,* 268:2984-2988). In assays of tissues or organs there will be less radioactivity from ¹²⁵I-KGF analog binding to KGFR, as compared to a standardized binding curve of ¹²⁵I-KGF analog, due to unlabeled native KGF binding to KGFR. Similarly, the use of ¹²⁵I-KGF analog may be used to detect the presence of KGFR in various cell types.

This invention also contemplates the use of a KGF analog in the generation of antibodies made against the peptide, which antibodies also bind to native KGF. In this embodiment, the antibodies are monoclonal or polyclonal in origin and are generated using a KGF analog. The resulting antibodies bind preferentially to native KGF, preferably when that protein is in its native (biologically active) conformation. These antibodies can be used for detection or purification of the native KGF.

Moreover, the invention contemplates the use of the KGF analogs in the discovery of high affinity or low affinity KGF binding molecules having therapeutical applications, for example, as a way for efficient KGF delivery or as an inhibitor for KGF activity. The thermal stability of the KGF analogs is important to identify such binding molecules in physiological conditions (i.e., at 37°C) since their affinity for KGF could be strongly temperature-dependent and may be unpredictable from the affinity observed at 4°C.

For *in vivo* uses, the KGF analogs may be formulated with additives. Such additives include buffers, carriers, stabilizers, excipients, preservatives, tonicity adjusting agents, anti-oxidants and the like (*e.g*., viscosity adjusting agents or extenders). The selection of specific additives will depend upon the storage form (i.e., liquid or lyophilized) and the modes of administering the KGF analog. Suitable formulations, known in the art, can be found in *REMINGTON'S PHARMACEUTICAL SCIENCES* (latest edition), Mack Publishing Company, Easton, PA.

The KGF analogs may be applied in therapeutically effective amounts to tissues specifically characterized by having damage to or clinically insufficient numbers of non-fibroblast epithelium cells. Areas in which KGF analogs may be successfully administered include, but are not limited to: the stimulation, proliferation and differentiation of adnexal structures such as hair follicles, sweat glands, and sebaceous glands in patients with burns and other partial and full-thickness injuries; accelerated reepithelialization of lesions caused by epidermolysis bullosa, which is a defect in adherence of the epidermis to the underlying dermis, resulting in frequent open, painful blisters which can cause severe morbidity; preventing chemotherapy-induced alopecia and treating male-pattern baldness, or the progressive loss of hair in men and women; treating gastric and duodenal ulcers; treating inflammatory bowel diseases, such a Crohn's disease (affecting primarily the small intestine) and ulcerative colitis (affecting primarily the large bowel); preventing or reducing gut toxicity in radiation and chemotherapy treatment regimes through treatment (e.g., pretreatment and/or postreatment) to induce a cytoprotective effect or regeneration or both; stimulating the production of mucus throughout the gastrointestinal tract; inducing the proliferation and differentiation of type II pneumocytes, which may help treat or prevent diseases such as hyaline membrane disease (i.e., infant respiratory distress syndrome and bronchopulmonary dysplasia) in premature infants; stimulating the proliferation and differentiation of the bronchiolar and/or alveolar epithelium with acute or chronic lung damage or insufficiency due to inhalation injuries (including high oxygen levels), emphysema, use of lung damaging chemotherapeutics, ventilator trauma or other lung damaging circumstances; increasing liver function to treat or prevent hepatic cirrhosis, fulminant liver failure, damage caused by acute viral hepatitis and/or toxic insults to the liver; inducing corneal cell regeneration, for example in the treatment of corneal abrasion; inducing epithelial cell regeneration to treat progressive gum disease; inducing regeneration of tympanic epithelial cells to treat ear drum damage and treating or preventing the onset of diabetes mellitus or as an adjunct in the setting of islet cell transplantation.

A patient in need of proliferation of non-fibroblast epithelial cells will be administered an effective amount of a KGF analog. An "effective amount" is that amount of the KGF analog required to elicit the desired response in the patient being treated and will, thus, generally be determined by the attending physician. Factors influencing the amount of KGF analog administered will include the age and general condition of the patient, the disease being treated, etc. Typical dosages will range from 0.001 mg/kg body weight to 500 mg/kg body weight.

The KGF analog may be safely administered parenterally (*e.g*., via IV, IT, IM, SC, or IP routes), orally or topically to warm-blooded animals (such as humans). The KGF analog may be used once or administered repeatedly, depending on the disease and the condition of the patient. In some cases, the KGF analog may be administered as an adjunct to other therapy and also with other pharmaceutical preparations.

The following examples are included to more fully illustrate the present invention.

### EXAMPLES

Standard methods for many of the procedures described in the following examples, or suitable alternative procedures, are provided in widely recognized manuals of molecular biology such as, for example, *Molecular Cloning,* Second Edition, Sambrook *et al.,* Cold Spring Harbor Laboratory Press (1987) and *Current Protocols in Molecular Biology,* Ausabel *et al.,* Greene Publishing Associates/Wiley Interscience, New York (1990).

### EXAMPLE 1: Preparation of DNA Coding for KGF and KGF Analogs

The cloning of the full-length human KGF gene (encoding a polypeptide with the sequence of native KGF) was carried out both by polymerase chain reaction (PCR) of RNA from an animal cell and by PCR of chemically synthesized (*E. coli* optimized codon) oligonucleotides ("OLIGOs"). Both procedures are described below:

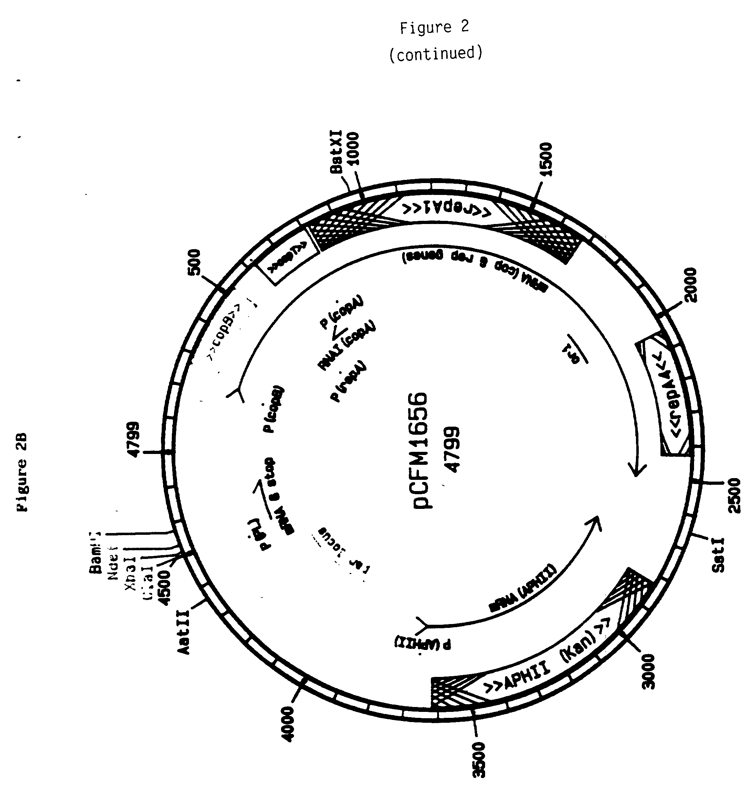

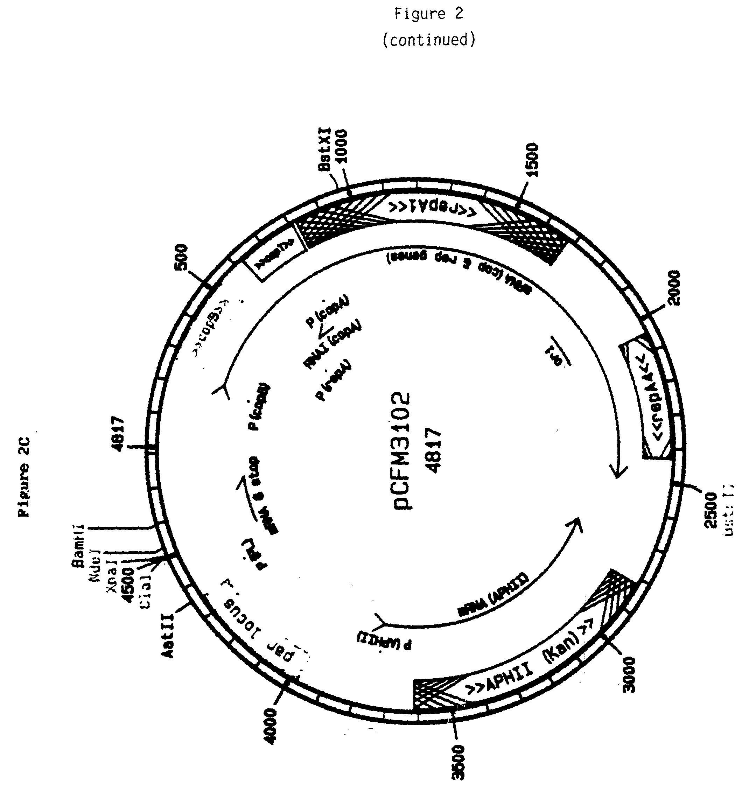

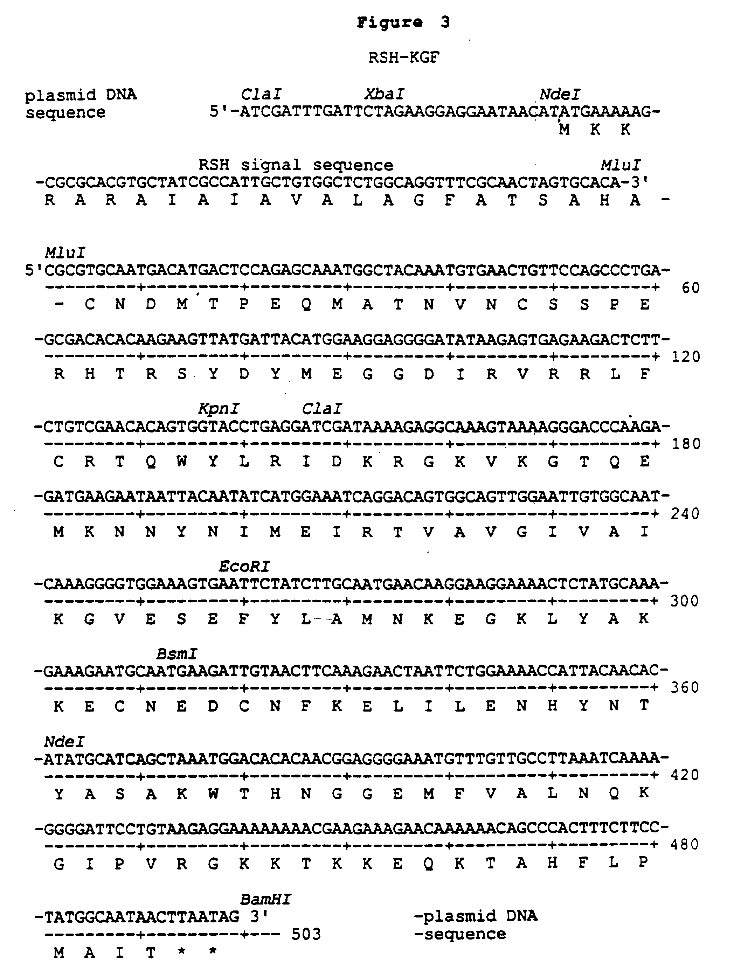

PCR amplification using RNA isolated from cells known to produce the polypeptide was performed. Initially, cells from a human fibroblast cell line AG1523A (obtained from Human Genetic Mutant Cell Culture Repository Institute For Medical Research, Camden, New Jersey) were disrupted with guanidium thiocyanate, followed by extraction (according to the method of Chomyzinski *et al.* (1987), *Anal. Biochem.,* 172:156). Using a standard reverse transcriptase protocol for total RNA, the KGF cDNA was generated. PCR (PCR#1) amplification of the KGF gene was carried out using the KGF cDNA as template and primers OLIGO#1 and OLIGO#2 that encode DNA sequences immediately 5' and 3' of the KGF gene [Model 9600 thermocycler (Perkin-Elmer Cetus, Norwalk, CT); 28 cycles; each cycle consisting of one minute at 94°C for denaturation, two minutes at 60°C for annealing, and three minutes at 72°C for elongation]. A small aliquot of the PCR#1 product was then used as template for a second KGF PCR (PCR#2) amplification identical to the cycle conditions described above except for a 50°C annealing temperature. For expression cloning of the KGF gene, nested PCR primers were used to create convenient restriction sites at both ends of the KGF gene. OLIGO#3 and OLIGO#4 were used to modify the KGF DNA product from PCR#2 to include *MluI* and *BamHI* restriction sites at the 5' and 3' ends of the gene, respectively [PCR#3; 30 cycles; each cycle consisting of one minute at 94°C for denaturation, two minutes at 60°C for annealing, and three minutes at 72°C for elongation]. This DNA was subsequently cut with *MluI* and *BamHI*, phenol extracted, and ethanol precipitated. It was then resuspended and ligated (using T4 ligase) into a pCFM1156 plasmid (Figure 2A) that contained a "RSH" signal sequence to make construct RSH-KGF (Figure 3).

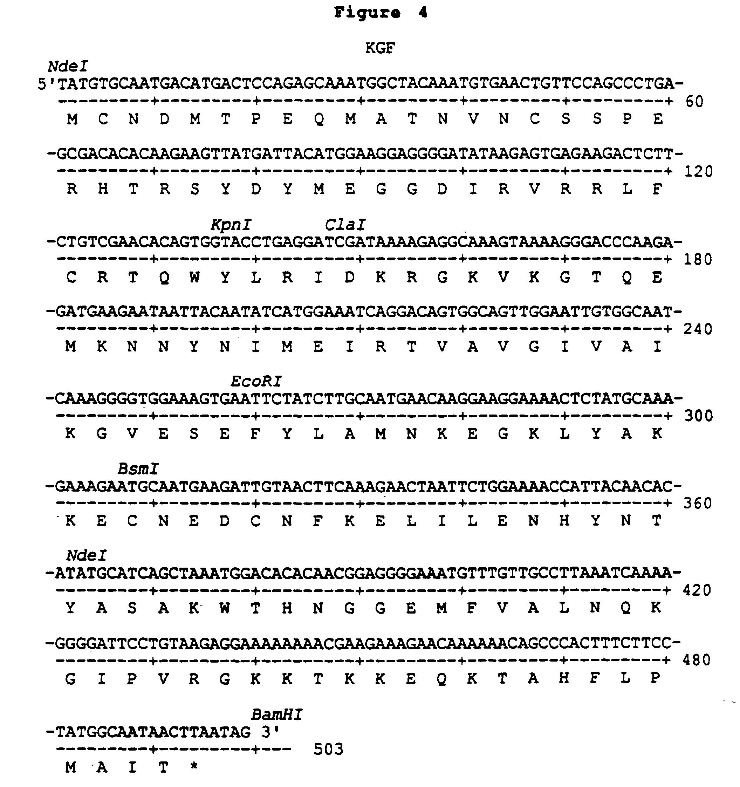

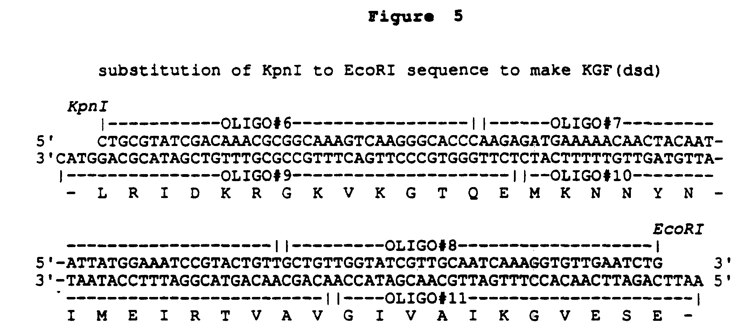

The ligation products were transformed (according to the method of Hanahan (1983), *J. Mol. Biol.,* 166:557) into *E. coli* strain FM5 (ATCC: 53911) and plated onto LB+kanamycin at 28°C. Several transformants were selected and grown in small liquid cultures containing 20 µg/mL kanamycin. The RSH-KGF plasmid was isolated from the cells of each culture and DNA sequenced. Because of an internal *NdeI* site in the KGF gene, it was not possible to directly clone the native gene sequence into the desired expression vector with the bracketed restriction sites of *NdeI* and *BamHI.* This was accomplished as a three-way ligation. Plasmid RSH-KGF was cut with the unique restriction sites of *BsmI* and *SstI,* and a ∼3 kbp DNA fragment (containing the 3' end of the KGF gene) was isolated following electrophoresis through a 1% agarose gel. A PCR (PCR#4) was carried out as described for PCR#3 except for the substitution of OLIGO#5 for OLIGO#3. The PCR DNA product was then cut with *NdeI* and *BsmI* and a 311 bp DNA fragment was isolated following electrophoresis through a 4% agarose gel. The third piece of the ligation is a 1.8 kbp DNA fragment of pCFM1156 cut with *NdeI* and *SstI* isolated following electrophoresis through a 1% agarose gel. Following ligation (T4 ligase), transformation, kanamycin selection and DNA sequencing as described above, a clone was picked containing the construct in Figure 4 and the plasmid designated KGF. Because of an internal ribosomal binding site that produced truncated products, the KGF DNA sequence between the unique *KpnI* and *EcoRI* sites was replaced with chemically synthesized OLIGOs (OLIGO#6 through OLIGO#11) to minimize the use of the internal start site (Figure 5).

OLIGO#1 (SEQ ID NO:7):     5'-CAATGACCTAGGAGTAACAATCAAC-3'
OLIGO#2 (SEQ ID NO:8):     5'-AAAACAAACATAAATGCACAAGTCCA-3'
OLIGO#3 (SEQ ID NO:9):     5'-ACAACGCGTGCAATGACATGACTCCA-3'
OLIGO#4 (SEQ ID NO:10):
          5'-ACAGGATCCTATTAAGTTATTGCCATAGGAA-3'
OLIGO#5 (SEQ ID NO:11):
          5'-ACACATATGTGCAATGACATGACTCCA-3'
OLIGO#6 (SEQ ID NO:12):
          5'-CTGCGTATCGACAAACGCGGCAAAGTCAAGGGCACCC-3'
OLIGO#7 (SEQ ID NO:13):
          5'-AAGAGATGAAAAACAACTACAATATTATGGAAATCCGTACTGTT-3'
OLIGO#8 (SEQ ID NO:14):
          5'-GCTGTTGGTATCGTTGCAATCAAAGGTGTTGAATCTG-3'
OLIGO#9 (SEQ ID NO:15):
          5'-TCTTGGGTGCCCTTGACTTTGCCGCGTTTGTCGATACGCAGGTAC-3'
OLIGO#10 (SEQ ID NO:16):
          5'-ACAGCAACAGTACGGATTTCCATAATATTGTAGTTGTTTTTCATC-3'
OLIGO#11 (SEQ ID NO:17):
          5'-AATTCAGATTCAACACCTTTGATTGCAACGATACCA-3'

The OLIGOs were phosphorylated with T4 polynucleotide kinase and then heat denatured. The single-stranded (ss) OLIGOs were then allowed to form a ds DNA fragment by allowing the temperature to slowly decrease to room temperature. T4 ligase was then used to covalently link both the internal OLIGO sticky-ends and the whole ds OLIGO fragment to the KGF plasmid cut with *KpnI* and *EcoRI*. The new plasmid was designated KGF(dsd).

A completely *E. coli* codon-optimized KGF gene was constructed by PCR amplification of chemically synthesized OLIGOs #12 through 24.

OLIGO#12 (SEQ ID NO:18): 5'-AGTTTTGATCTAGAAGGAGG-3'
OLIGO#13 (SEQ ID NO:19): 5'-TCAAAACTGGATCCTATTAA-3'
OLIGO#14 (SEQ ID NO:20):
5'-AGTTTTGATCTAGAAGGAGGAATAACATATGTGCAACGACATG-
ACTCCGGAACAGATGGCTACCAACGTTAACTGCTCCAGCCCGGAACGT-3'
OLIGO#15 (SEQ ID NO:21):
5'-CACACCCGTAGCTACGACTACATGGAAGGTGGTGACATCCGT-
GTTCGTCGTCTGTTCTGCCGTACCCAGTGGTACCTGCGTATCGACAAA-3'
OLIGO#16 (SEQ ID NO:22):
5'-CGTGGTAAAGTTAAAGGTACCCAGGAAATGAAAAACAACTACAACATC-
ATGGAAATCCGTACTGTTGCTGTTGGTATCGTTGCAATCAAA-3'
OLIGO#17 (SEQ ID NO:23):
5'-GGTGTTGAATCTGAATTCTACCTGGCAATGAACAAAGAAGGTAAACT-
GTACGCAAAAAAAGAATGCAACGAAGACTGCAACTTCAAAGAA-3'
OLIGO#18 (SEQ ID NO:24):
5'-CTGATCCTGGAAAACCACTACAACACCTACGCATCTGCTAAATGGAC-
CCACAACGGTGGTGAAATGTTCGTTGCTCTGAACCAGAAAGGT-3'
OLIGO#19 (SEQ ID NO:25):
5'-ATCCCGGTTCGTGGTAAAAAAACCAAAAAGAACAGAAAACCGCTC-
ACTTCCTGCCGATGGCAATCACTTAATAGGATCCAGTTTTGA-3'
OLIGO#20 (SEQ ID NO:26):5'-TACGGGTGTGACGTTCCGGG-3'
OLIGO#21 (SEQ ID NO:27):5'-CTTTACCACGTTTGTCGATA-3'
OLIGO#22 (SEQ ID NO:28):5'-ATTCAACACCTTTGATTGCA-3'
OLIGO#23 (SEQ ID NO:29):5'-CCAGGATCAGTTCTTTGAAG-3'
OLIGO#24 (SEQ ID NO:30):5'-GAACCGGGATACCTTTCTGG-3'

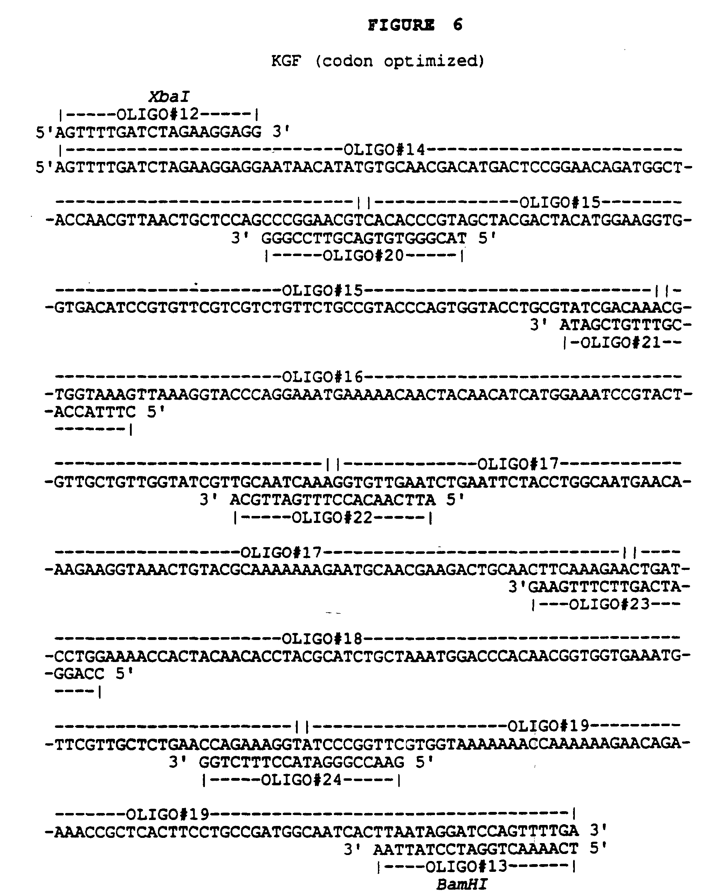

OLIGOs #12 through 24 were designed so that the entire DNA sequence encoding native KGF was represented by OLIGOs from either the "Watson" or the "Crick" strand and upon PCR amplification would produce the desired double-stranded DNA sequence (Figure 6) [PCR#5, Model 9600 thermocycler, Perkin-Elmer Cetus]; 21 cycles, each cycle consisting of 31 seconds at 94°C for denaturation, 31 seconds at 50°C for annealing, and 31 seconds at 73°C for elongation; following the 21 cycles the PCR was finished with a final elongation step of 7 minutes]. After PCR amplification, the DNA fragment was cut with *XbaI* and *BamHI* and the 521 bp fragment ligated into the expression plasmid pCFM1156 cut with the same enzymes. PCR#5 utilized the outside primers (100 pmoles/100 µl rxn) OLIGO#12 and OLIGO#13 and 1 µl/100 µl rxn of a KGF template derived by ligation (by T4 ligase) of OLIGO#14 through OLIGO#19 (OLIGO#15 through OLIGO#18 were phosphorylated with T4 polynucleotide kinase) using OLIGO#20 through OLIGO#24 as band-aid oligos (Jayaraman *et al*. (1992), *Biotechniques,* 12:392) for the ligation. The final construct was designated KGF (codon optimized).

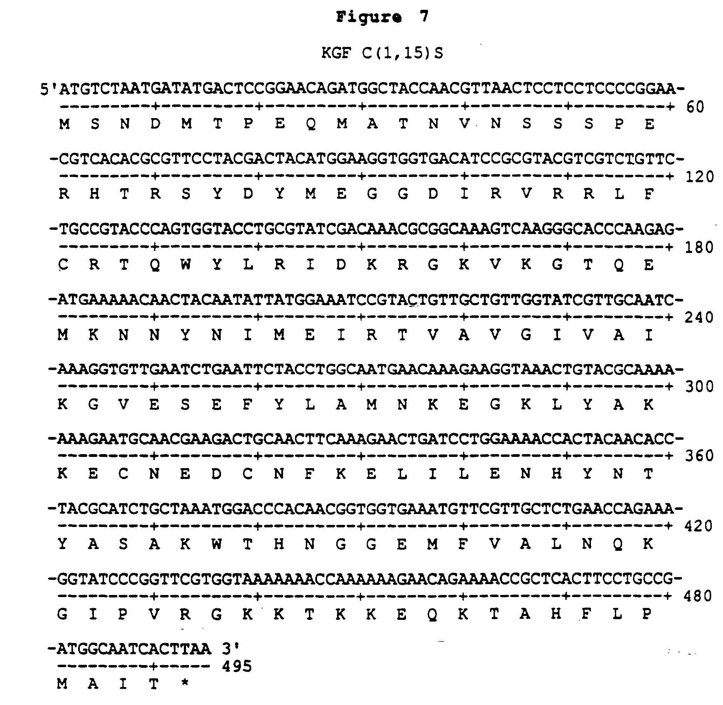

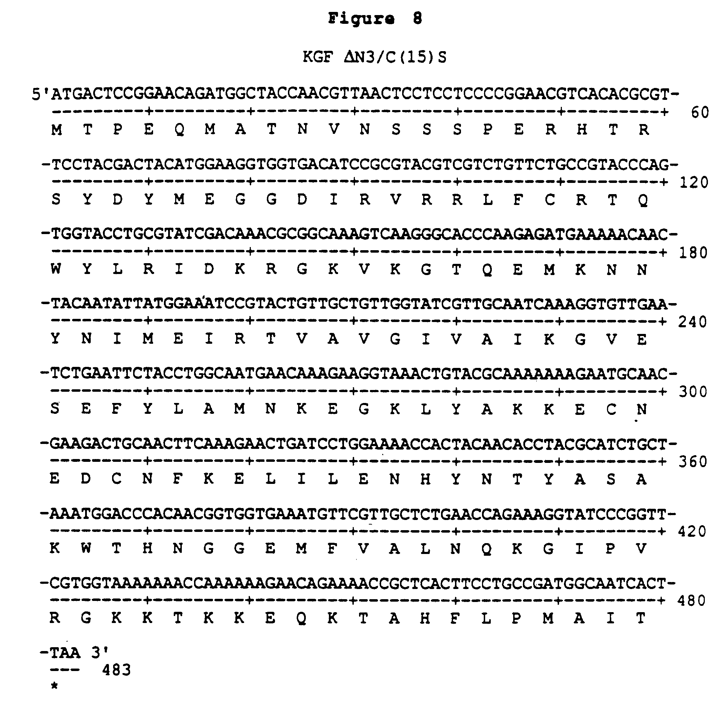

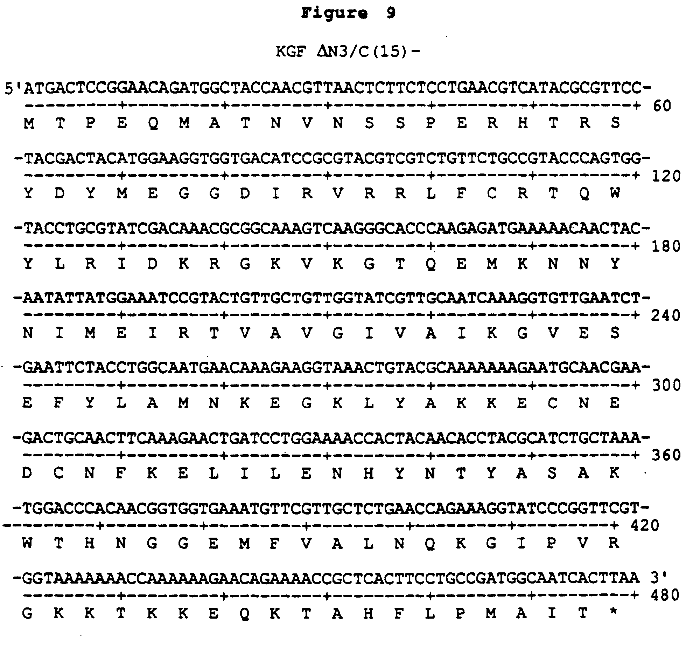

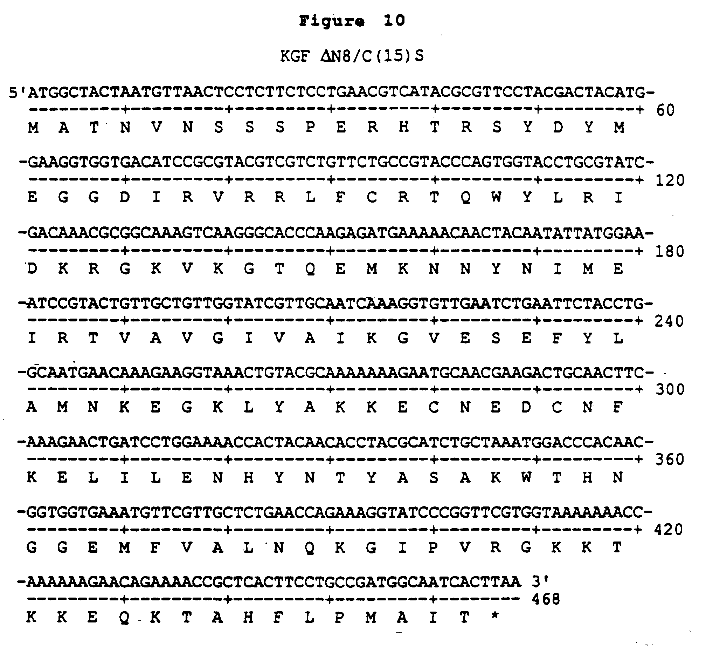

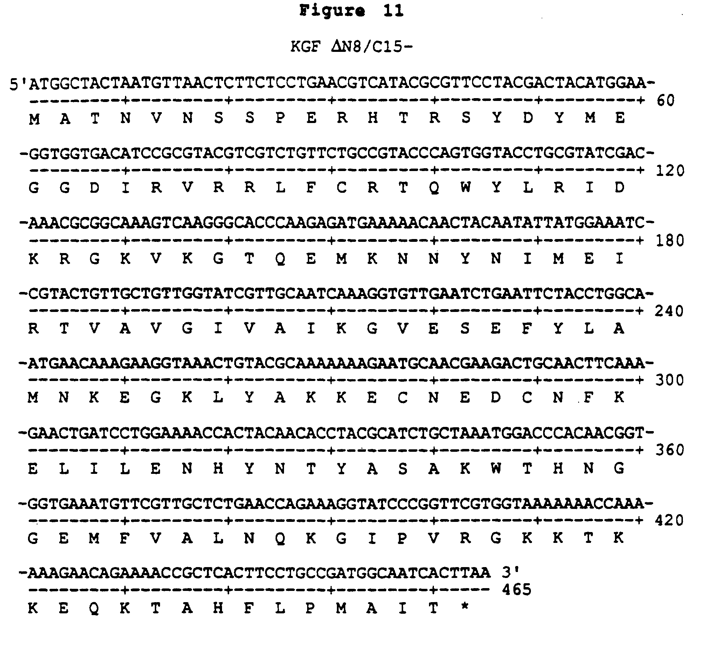

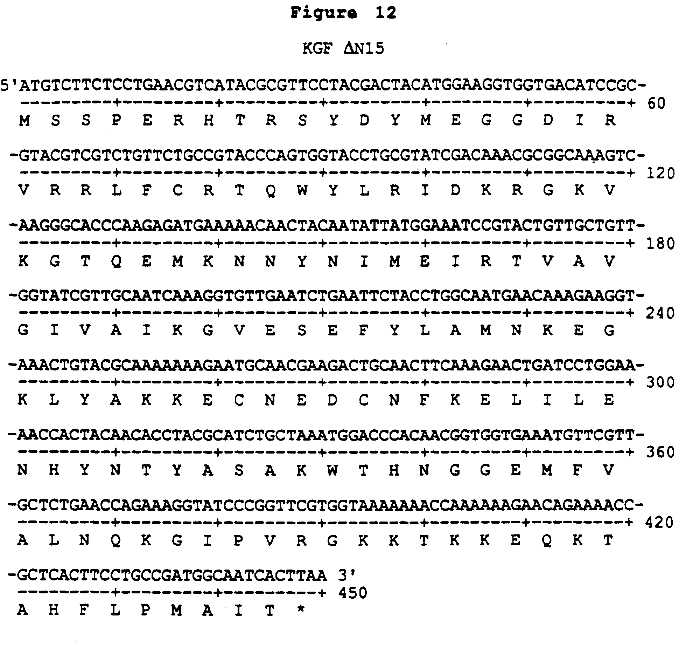

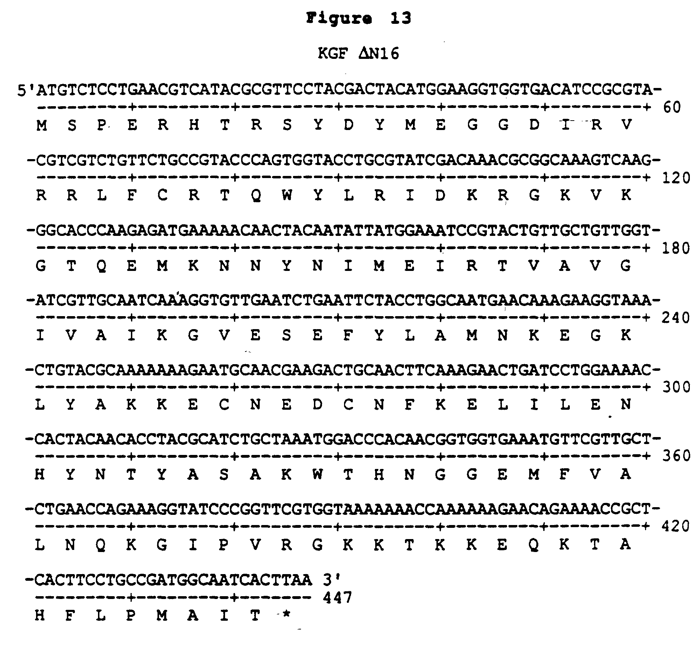

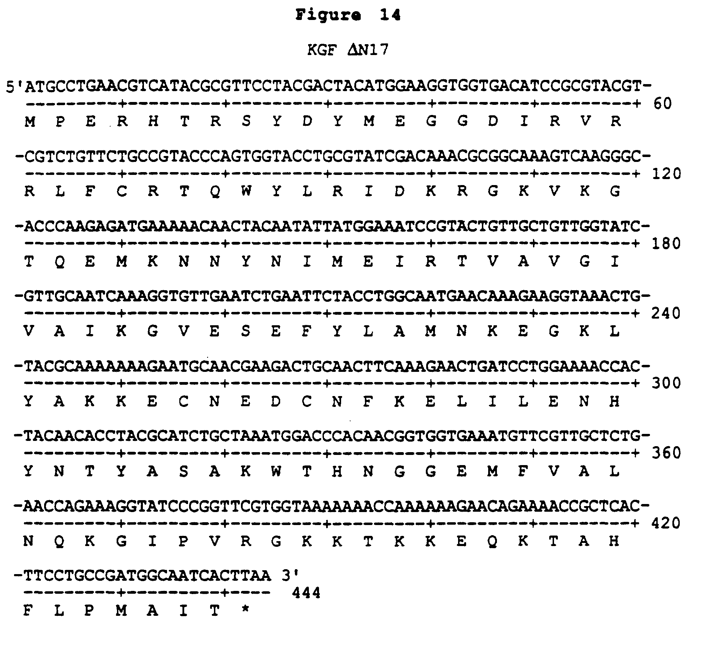

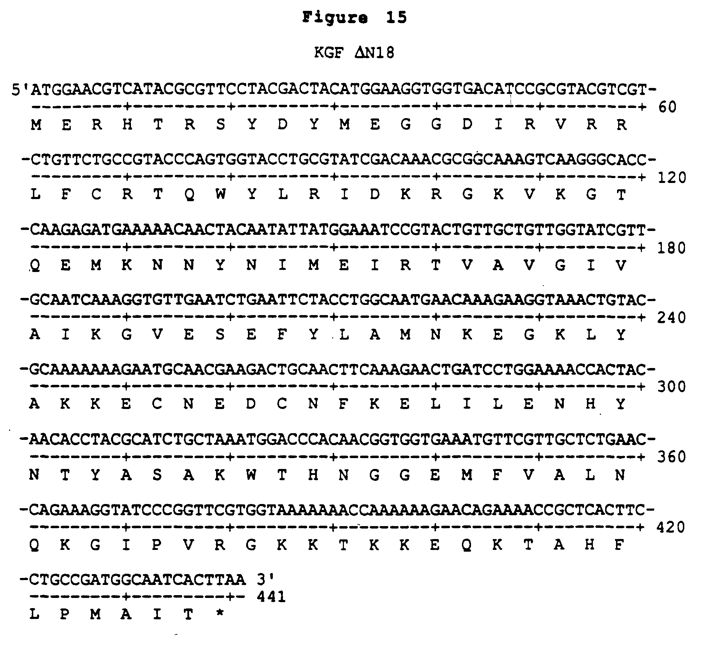

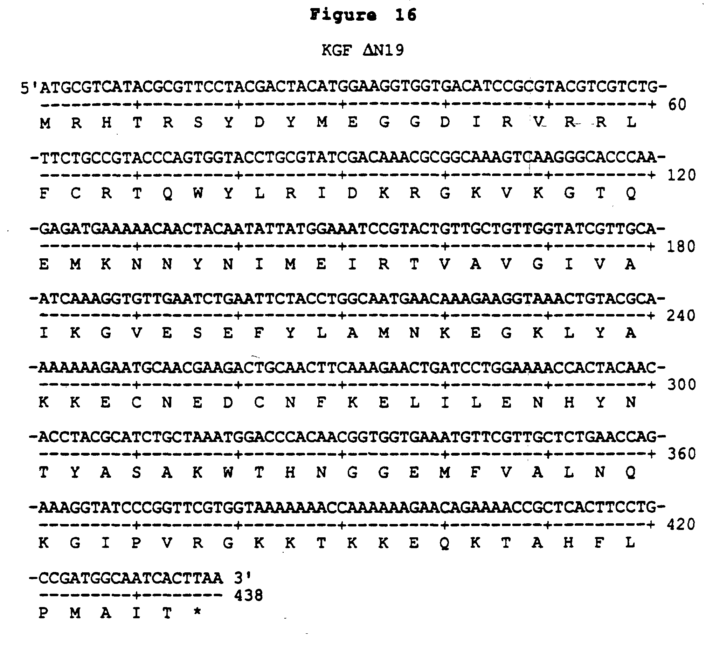

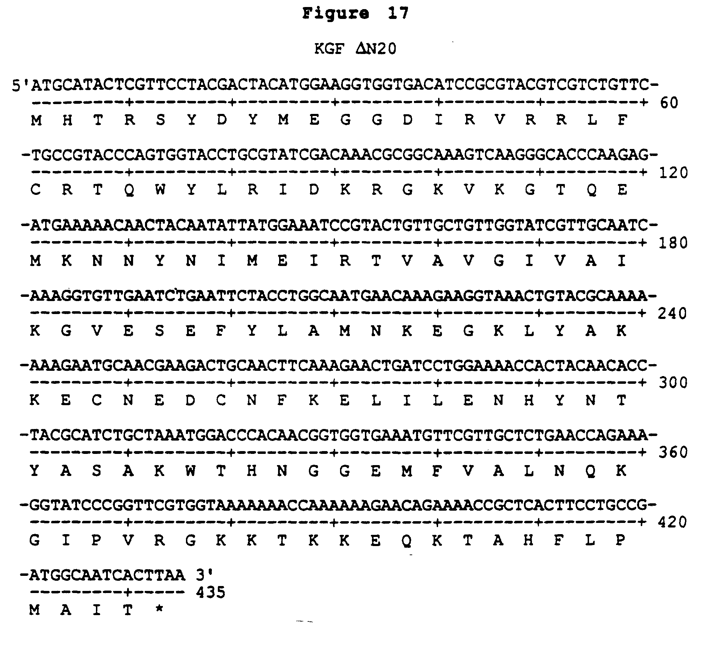

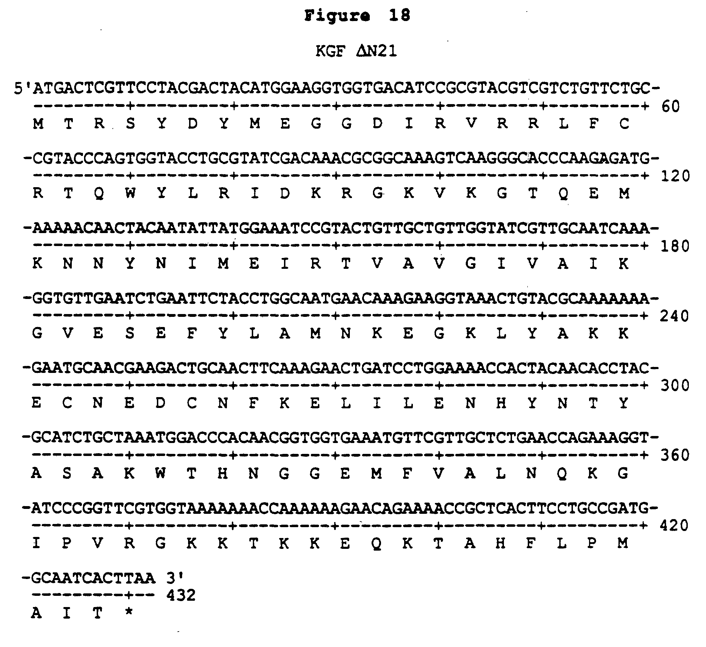

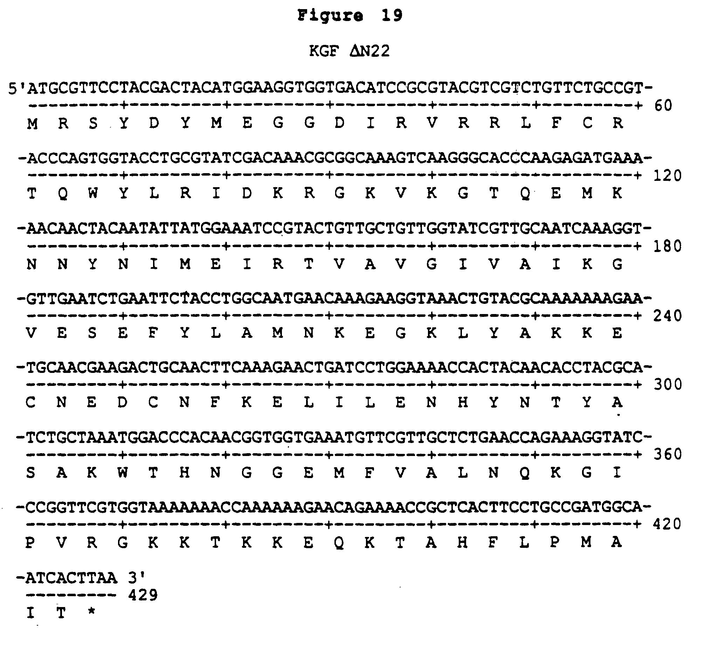

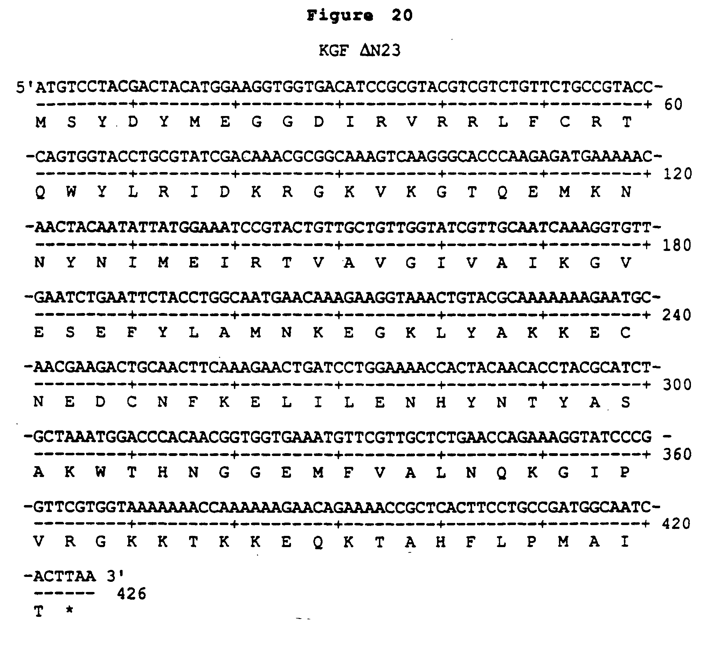

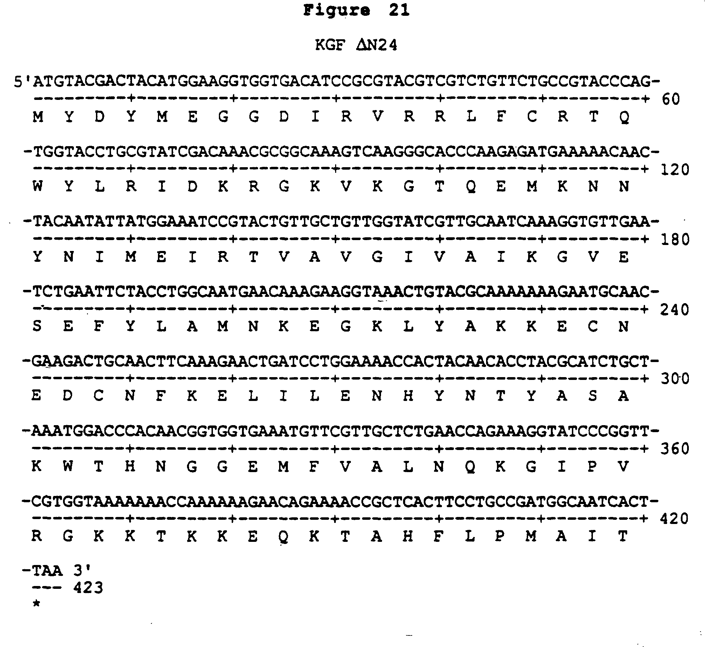

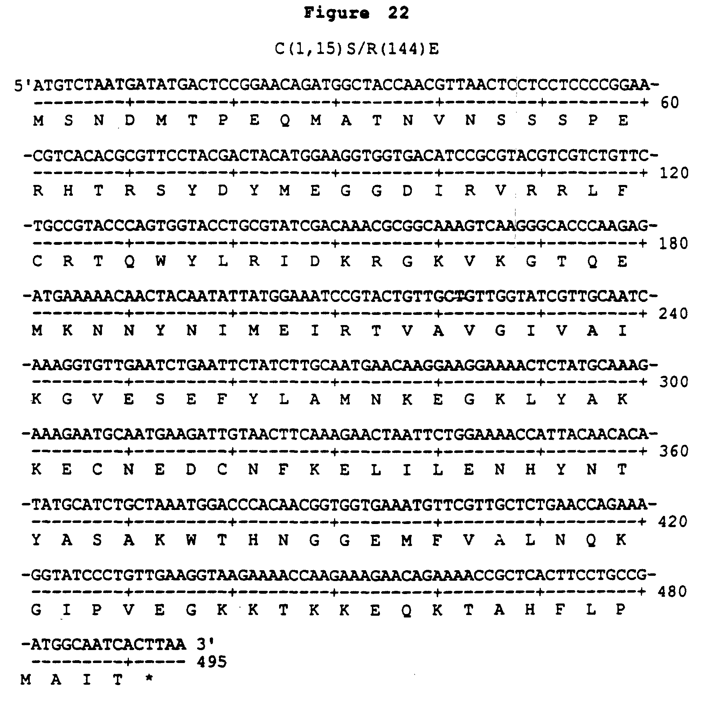

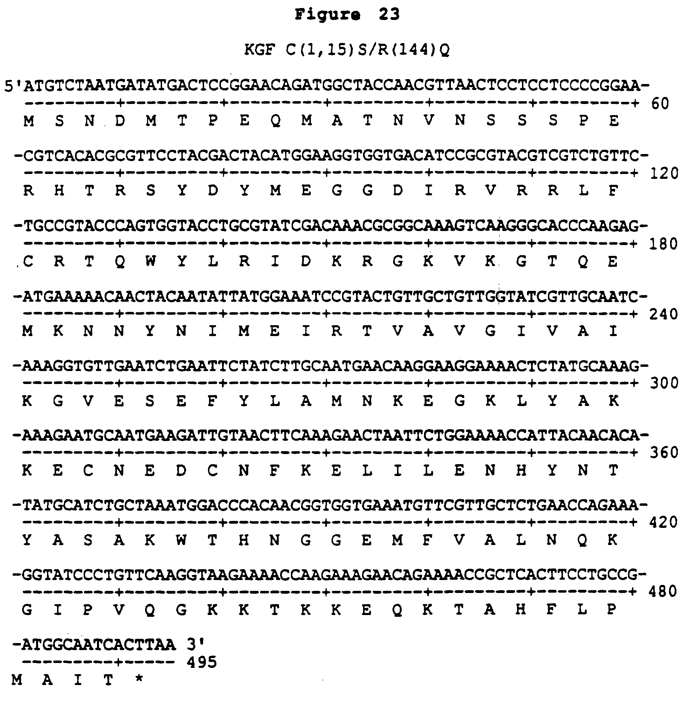

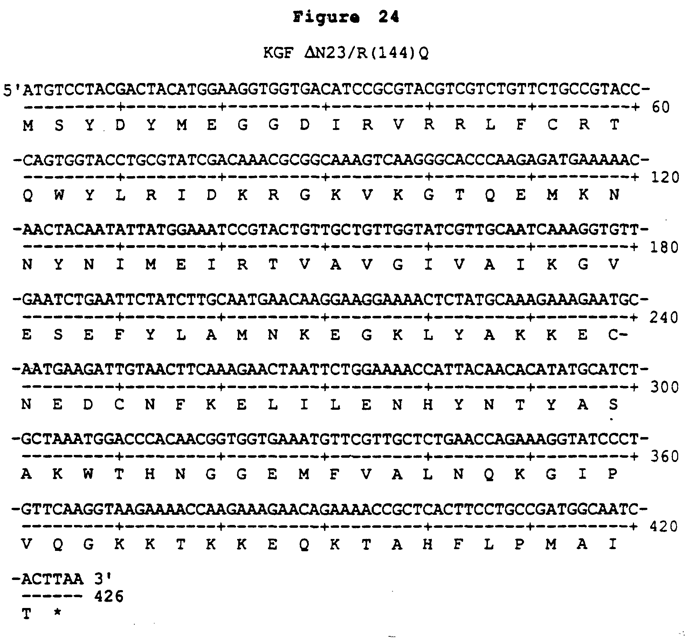

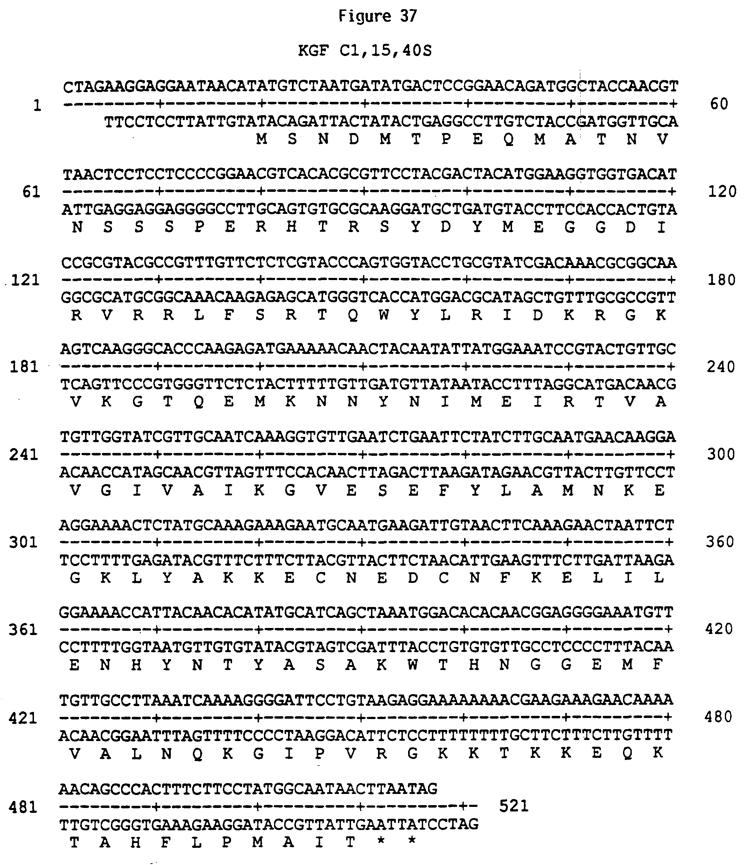

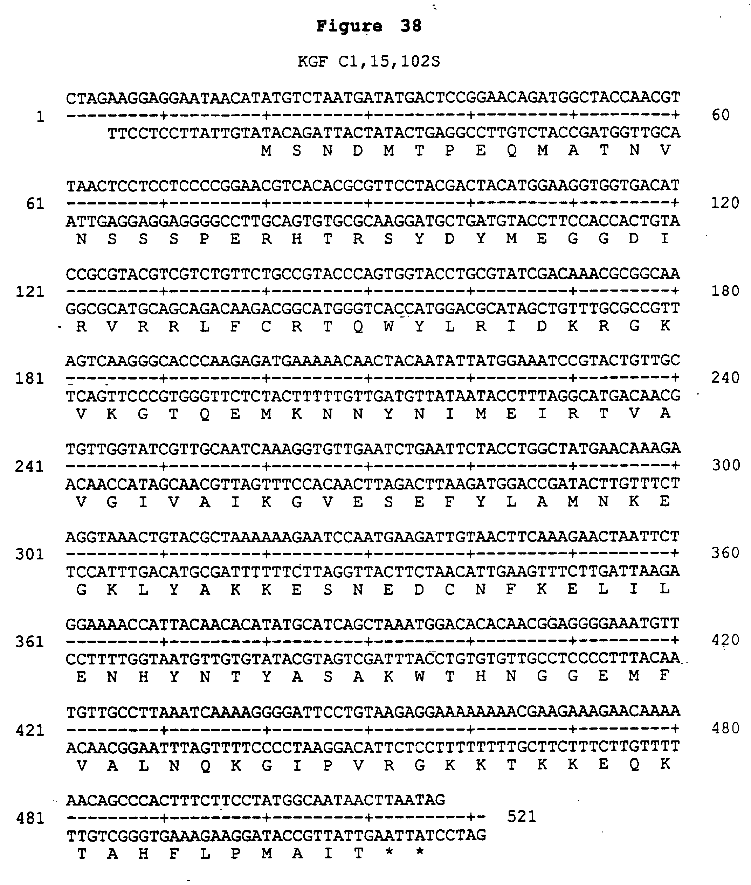

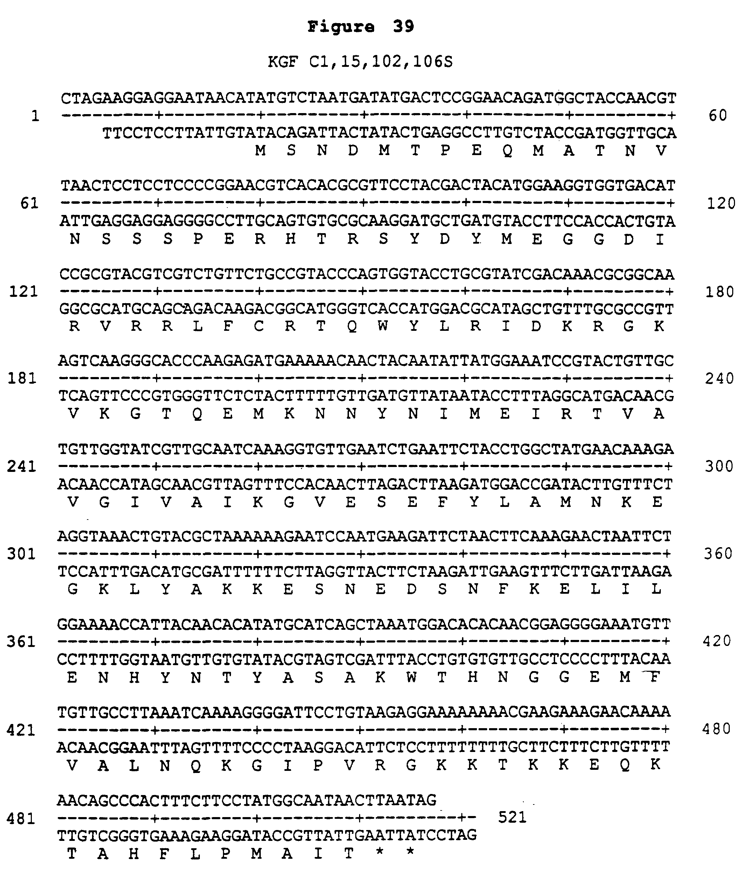

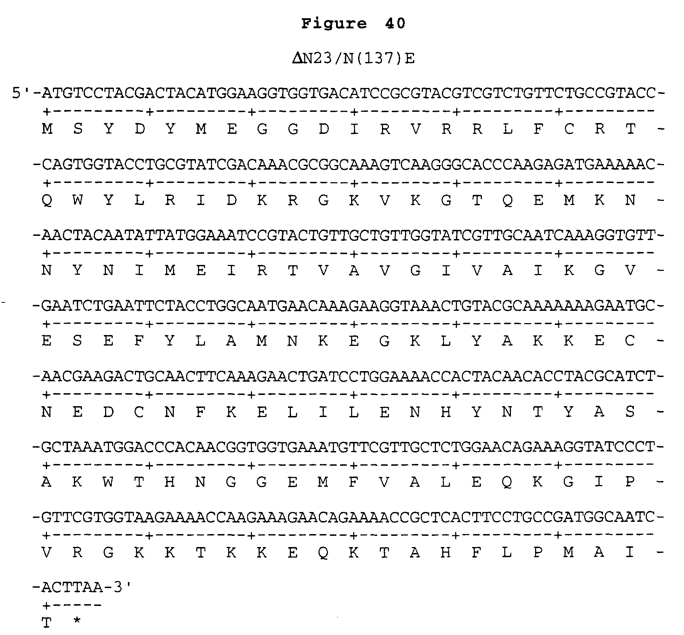

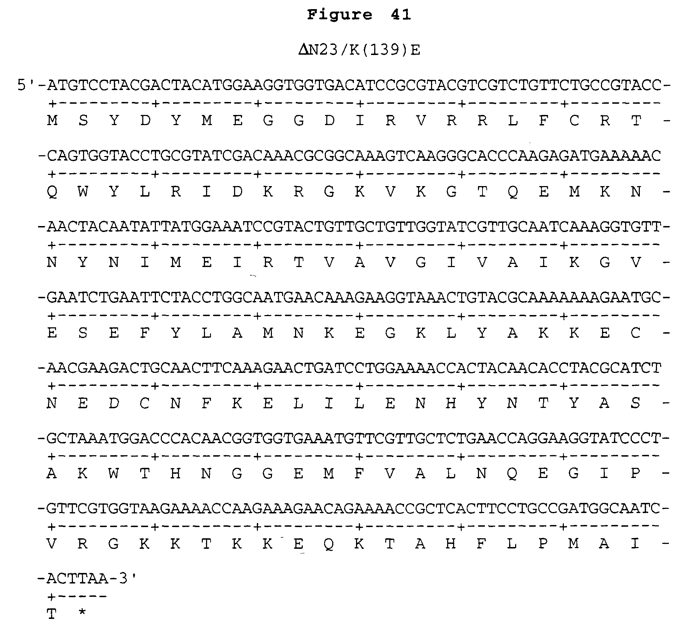

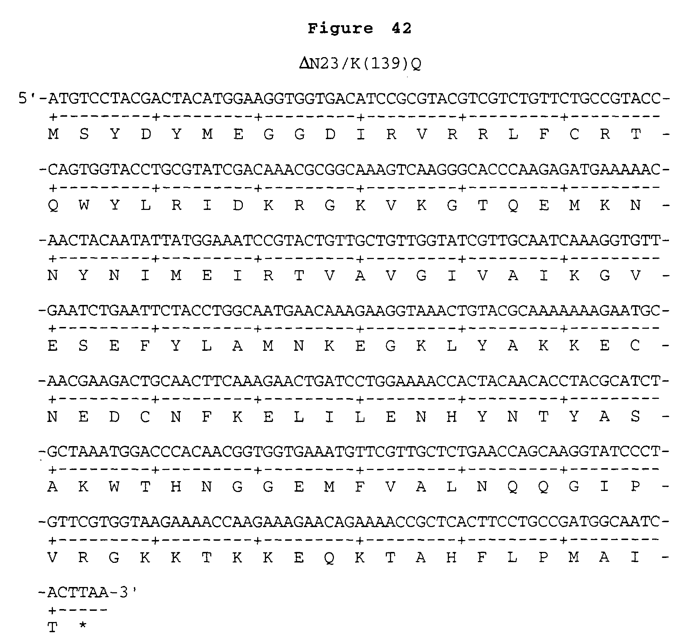

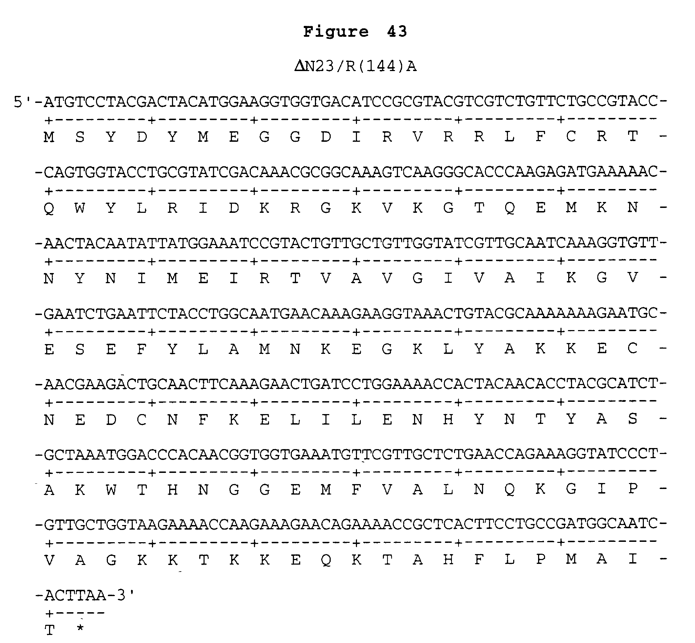

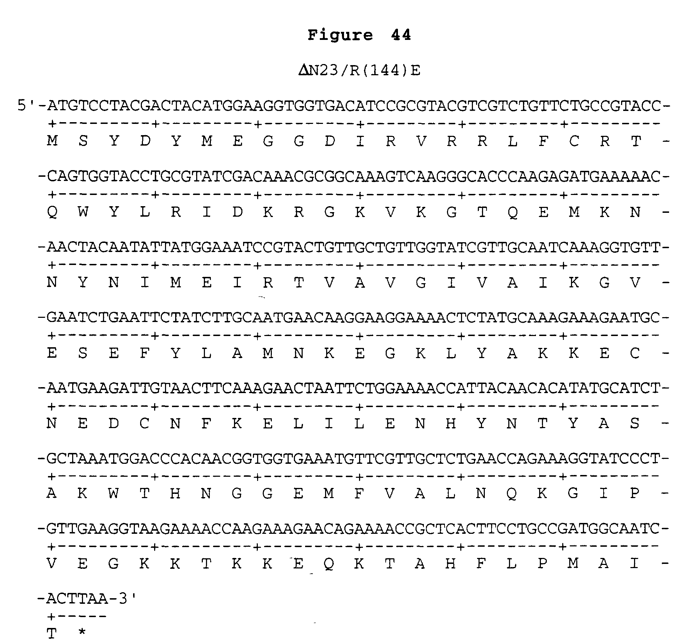

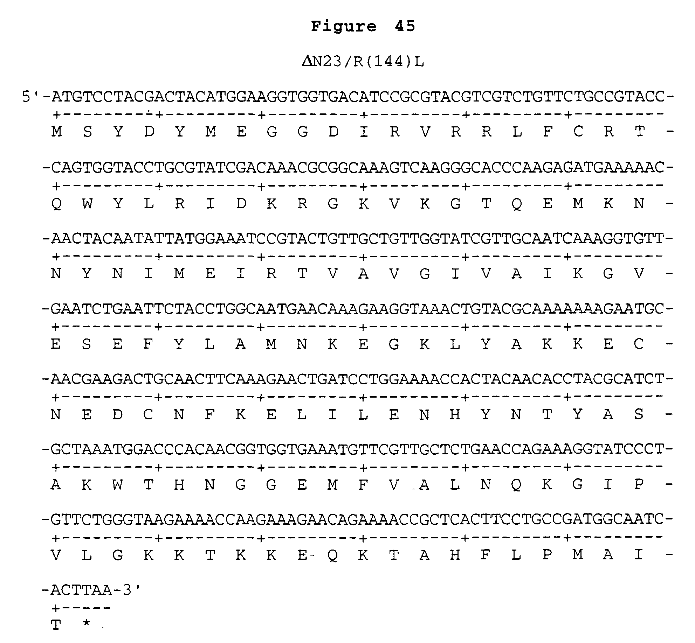

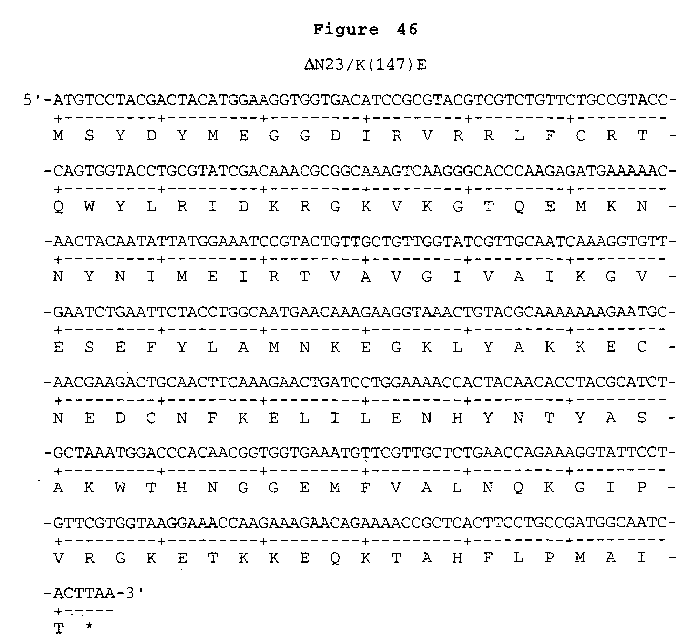

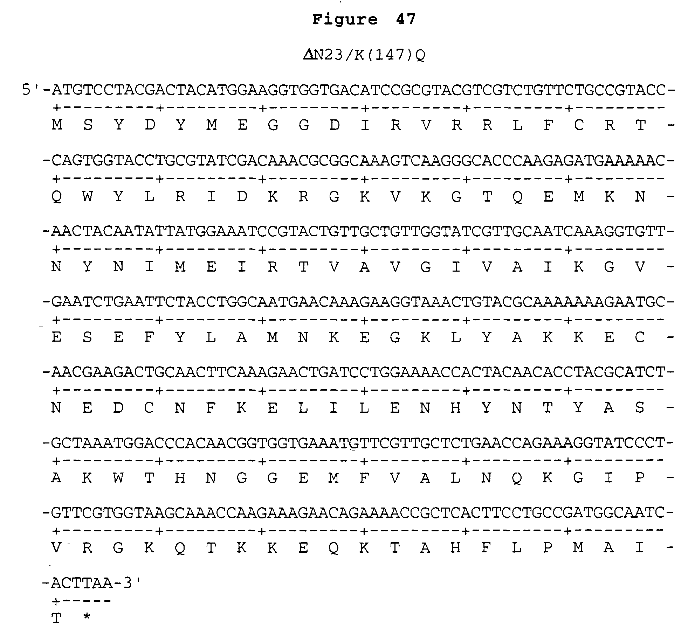

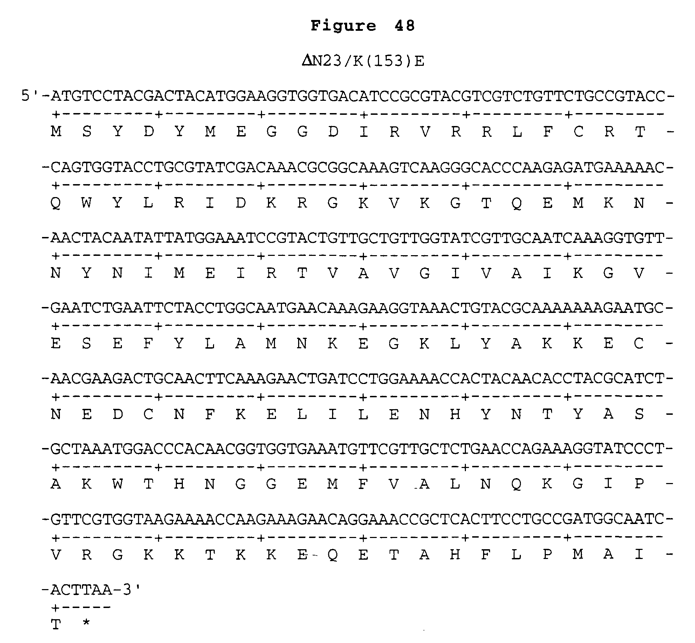

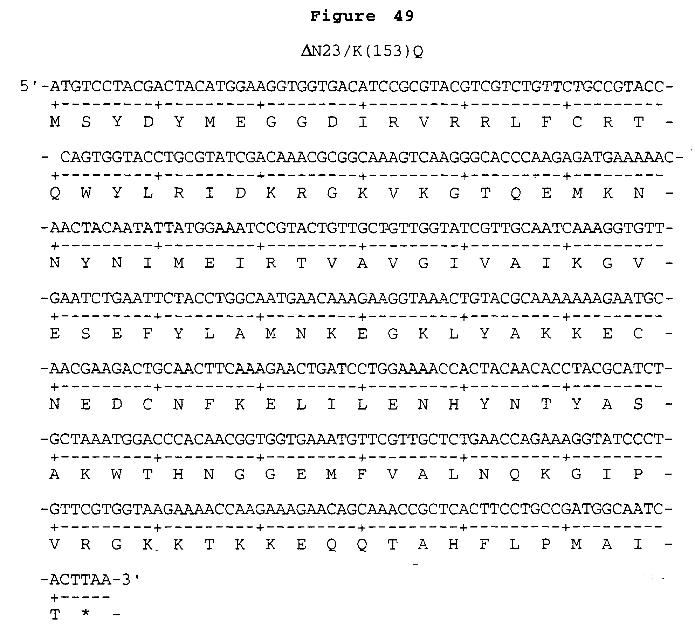

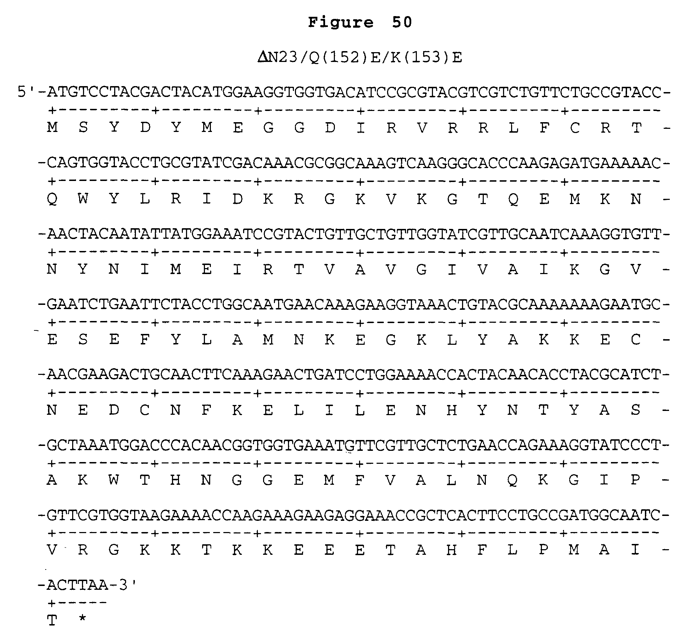

All of the KGF analogs described herein are composed in part from DNA sequences found in KGF(dsd) or KGF(codon optimized), or a combination of the two. The sequences are further modified by the insertion into convenient restriction sites of DNA sequences that encode the particular KGF analog amino acids made utilizing one or more of the above-described techniques for DNA fragment synthesis. Any of the analogs can be generated in their entirety by the above described techniques. However, as a part of the general OLIGO design optimized *E. coli* codons were used where appropriate, although the presence of *E. coli* optimized codons in part or *in toto* of any of the genes where examined did not significantly increase the yield of protein that could be obtained from cultured bacterial cells. Figures 7 to 24 and 37 to 50 set forth by convenient example particular KGF analog nucleotide and amino acid sequence constructions: C(1,15)S (Figure 7); ΔN3/C(15)S (Figure 8); ΔN3/C(15)- (Figure 9); ΔN8/C(15)S (Figure 10); ΔN8/C(15)- (Figure 11); ΔN15 (Figure 12); ΔN16 (Figure 13); ΔN17 (Figure 14); ΔN18 (Figure 15); ΔN19 (Figure 16); ΔN20 (Figure 17); ΔN21 (Figure 18); ΔN22 (Figure 19); ΔN23 (Figure 20); ΔN24 (Figure 21); C(1,15)S/R(144)E (Figure 22); C(1,15)S/R(144)Q (Figure 23); ΔN23/R(144)Q (Figure 24); C(1,15,40)S (Figure 37); C(1,15,102)S (Figure 38); C(1,15,102,106)S (Figure 39); ΔN23/N(137)E (Figure 40); ΔN23/K(139)E (Figure 41); ΔN23/K(139)Q (Figure 42); ΔN23/R(144)A (Figure 43); ΔN23/R(144)E (Figure 44); ΔN23/R(144)L (Figure 45); ΔN23/K(147)E (Figure 46); ΔN23/K(147)Q (Figure 47); ΔN23/K(153)E (Figure 48); ΔN23/K(153)Q (Figure 49) and ΔN23/Q(152)E/K(153)E (Figure 50). All the KGF analog constructions described herein were DNA sequence confirmed.

### EXAMPLE 2: Production in E. coli

### A. Expression of KGF Analogs

Three different expression plasmids were utilized in the cloning of the KGF analog genes. They were pCFM1156 (ATCC# 69702), pCFM1656 (ATCC# 69576), and pCFM3102 (Figures 2A, 2B and 2C, respectively). The plasmid p3102 can be derived from the plasmid pCFM1656 by making a series of site-directed base changes with PCR overlapping oligo mutagenesis. Starting with the *BglII* site (pCFM1656 plasmid bp # 180) immediately 5' to the plasmid replication promoter, PcopB, and proceeding toward the plasmid replication genes, the base pair changes are as follows:

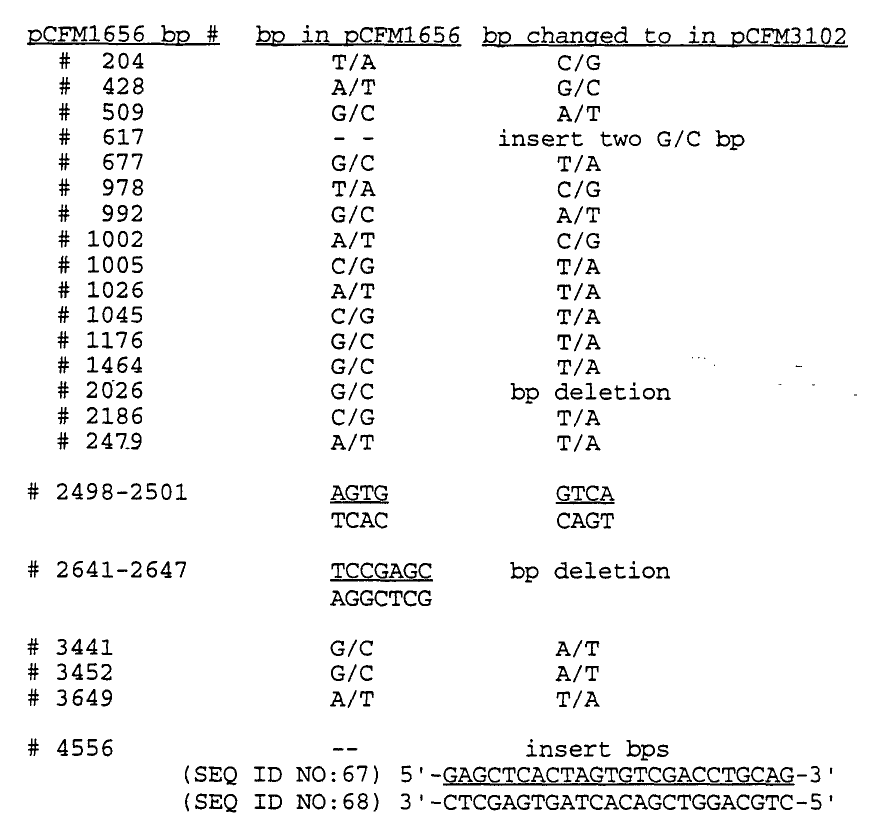

As seen above, pCFM1156, pCFM1656 and pCFM3102 are very similar to each other and contain many of the same restriction sites. The plasmids were chosen by convenience, and the vector DNA components can be easily exchanged for purposes of new constructs. The host used for all cloning was *E. coli* strain FM5 (ATCC: 53911) and the transformations were carried out (according to the method of Hanahan (1983), *supra)* or by electroelution with a Gene Pulser™ transfection apparatus (BioRad Laboratories, Inc., Hercules, CA) according to the manufacturer's instructions.

Initially, a small, freshly cultured inoculum of the desired recombinant *E. coli* clone harboring the desired construct on one of the three pCFM vectors was started by transferring 0.1 mL of a frozen glycerol stock of the appropriate strain into a 2 L flask containing 500 mL of Luria broth. The culture was shaken at 30°C for 16 hours, after which the culture was transferred to a 15 L fermentor containing 8 L of sterile batch medium (Tsai, *et al.* (1987), *J. Industrial Microbiol.,* 2:181-187).

Feed batch fermentation starts with the feeding of Feed # 1 medium (Tsai, *et al.* (1987), *supra).* When the OD600 reached 35, expression of the desired KGF analog was induced by rapidly raising the culture temperature to 37°C for two hours then up to 42°C to denature the CI repressor. The addition of Feed 1 was discontinued in favor of Feed 2, the addition rate of which was initiated at 300 mL/hr. Feed 2 comprised 175 g/L trypticase-peptone, 87.5 g/L yeast extract, and 260 g/L glucose. After one hour at 42'C, the culture temperature was decreased to 36°C, where this temperature was then maintained for another 6 hours.

The fermentation was then halted and the cells were harvested by centrifugation into plastic bags placed within 1 L centrifuge bottles. The cells were pelleted by centrifugation at 400 rpm for 60 minutes, after which the supernatants were removed and the cell paste frozen at -90°C.

Following expression of the various KGF analogs in *E. coli,* native KGF, C(1,15)S, C(1,15)S/R(144)E, C(1,15)S/R(144)Q, ΔN15, ΔN23, and ΔN23/R(144)Q proteins were purified using the following procedure. Cell paste from a high cell density fermentation was suspended at 4°C in 0.2 M NaCl, 20 mM NaPO₄, pH 7.5 as a 10-20% solution (weight per volume) using a suitable high shear mixer. The suspended cells were then lysed by passing the solution through a homogenizer (APV Gaulin, Inc., Everett, MA) three times. The outflowing homogenate was cooled to 4-8°C by using a suitable heat exchanger. Debris was then removed by centrifuging the lysate in a J-6B™ centrifuge (Beckman Instruments, Inc., Brea, CA) equipped with a JS 4.2 rotor at 4,200 rpm for 30-60 min. at 4°C. Supernatants were then carefully decanted and loaded onto a previously prepared 450 mL (5 cm x 23 cm) column of S-Sepharose Fast Flow™ resin (Pharmacia, Piscataway, NJ) equilibrated with 0.2 M NaCl, 20 mM NaPO₄, pH 7.5 at 4°C. Next, the column was washed with five column volumes (2250 mL) of 0.4 M NaCl, 20 mM NaPO₄, pH 7.5 at 4°C. The desired protein was eluted by washing the column with 5 L of 0.5 M NaCl, 20 mM NaPO₄, pH 7.5. 50 mL fractions were collected and the A₂₈₀ of the effluent was continuously monitored. Fractions identified by A₂₈₀ as containing eluted material were then analyzed by SDS-PAGE through 14% gels to confirm the presence of the desired polypeptide.

Those fractions containing proteins of interest were then pooled, followed by the addition of an equal volume of distilled water. The diluted sample was then loaded onto a previously prepared a 450 mL (5 cm x 23 cm) column of S-Sepharose Fast Flow equilibrated with 0.4 M NaCl, 20 mM NaPO₄, pH 6.8 at 4°C. The column was washed with 2250 mL of 0.4 M NaCl, 20 mM NaPO₄, pH 6.8 and the protein eluted using a 20 column volume linear gradient ranging from 0.4 M NaCl, 20 mM NaPO₄, pH 6.8 to 0.6 M NaCl, 20 mM NaPO₄, pH 6.8. Again, 50 mL fractions were collected under constant A₂₈₀ monitoring of the effluent. Those fractions containing the protein (determined by 14% SDS-PAGE) were then pooled, followed by concentration through a YM-10 membrane (10,000 molecular weight cutoff) in a 350cc stirring cell (Amicon, Inc. Mayberry, MA) to a volume of 30-40 mL.

The concentrate was then loaded onto a previously generated 1,300 mL (4.4 cm x 85 cm) column of Superdex-75™ resin (Pharmacia) equilibrated in column buffer comprising 1X PBS (Dulbecco's Phosphate Buffered Saline, "D-PBS", calcium and magnesium-free) or 0.15 M NaCl, 20 mM NaPO₄, pH 7.0. After allowing the sample to run into the column, the protein was eluted from the gel filtration matrix using column buffer. Thereafter, 10 mL fractions were recovered and those containing the analog (determined by 14% SDS-PAGE) were pooled. Typically, the protein concentration was about 5-10 mg/mL in the resultant pool. All of the above procedures were performed at 4-8°C, unless otherwise specified.

An alternative purification procedure was used to purify native KGF, C(1,15)S and ΔN23. The procedure involves the following steps, and unless otherwise specified, all procedures, solutions and materials were conducted at 23 ± 5°C.

Upon completion of the production phase of a bacterial fermentation, the cell culture was cooled to 4-8°C and the cells harvested by centrifugation or a similar process. On the basis of the expected yield of protein per unit weight of cell paste and the amount of purified protein required, an appropriate amount of cell paste, by weight, was suspended in a mild buffer solution of 20 mM NaPO₄, 0.2 M NaCl, pH 7.5, weighing about five times that of the cell paste to be suspended. The cells were dispersed to a homogeneous solution using a high shear mixer. The temperature of the cell paste dispersion was maintained at 4-8°C during homogenization.

The cells were then lysed by pressure, for example by passing the cell paste dispersion twice through an appropriately-sized cell homogenizer. The homogenate was kept chilled at 5 ± 3°C. To clarify the cell lysate, a previously prepared depth filter housing (Cuno, Inc., Meriden, CT) equipped with a filter having an appropriate amount of filter surface area, equilibrated with a suitable volume of 0.2 M NaCl, 20 mM NaPO₄, pH 7.5, was employed. The equilibration and clarification were performed at 5 ± 3°C. Prior to clarification, an appropriate amount of a suitable filter aid was used to pre-coat the filter and be thoroughly mixed with the cell lysate, after which the lysate was clarified by passing the solution through the filter apparatus. The filter was washed with 0.2 M NaCl, 20 mM NaPO₄, pH 7.5. The filtrate and any subsequent wash were collected in a chilled container of suitable capacity, all the while being maintained at less than 10°C.

Following clarification, the lysate was then passed through a previously prepared a column of SP-Sepharose Fast Flow containing at least 1 mL of resin per 2 g of cell paste. The column of SP-Sepharose Fast Flow was equilibrated with cold (5 ± 3°C), 0.2 M NaCl, 20 mM NaPO₄, pH 7.5. The temperature of the column was maintained at less than 10°C. The clarified lysate (5 ± 3°C) was then loaded onto the ion exchange column, with the absorbance at 280 nm (A₂₈₀) of eluate being continuously monitored. After sample loading, the column was washed with cold 0.2 M NaCl, 20 mM NaPO₄, pH 7.5, followed by washing with 0.3 M NaCl, 20 mM NaPO₄, pH 7.5 at 23 ± 5°C.

To elute the desired protein, a linear gradient ranging from 0.2-1 M NaCl, 20 mM NaPO₄, pH 7.5 was used. Bulk product was collected in several fractions on the basis of the A₂₈₀ of the eluate. Following elution, these fractions were pooled and the volume noted.

To oxidize free sulfhydryl groups, an oxidation step was performed. For proteins with altered cysteine patterns, as compared to native KGF, an oxidizing agent (e.g., cystamine dihydrochloride or another appropriate oxidizing agent, for instance, cystine, oxidized glutathione or divalent copper) was added to a final concentration of 1-20 mM and the pH was adjusted to 7-0.5, with a pH of 9.0 ± 0.3 being preferred when cystamine dihydrochloride was used. The oxidation was conducted at 10-30°C for an appropriate period. For the native KGF protein, oxidation was accomplished by adding an appropriate amount of (NH₄)₂SO₄ such as 1-2 M (NH₄)₂SO₄, adjusting the pH to 7.5 ± 0.5, and holding the temperature at 23 ± 5°C for an appropriate period.

After oxidation, the pH of the solution was adjusted to between 6.5 and 9.5. If necessary, solid (NH₄)₂SO₄ was added to the solution to a final concentration of 2 M. To remove particulates, the solution was passed through appropriate clarification filters.

The filtered, oxidized product was then subjected to hydrophobic interaction chromatography (HIC). The HIC matrice was Butyl-650M Toyopearl™ resin (Tosohaas, Inc., Montgomeryville, PA). The protein-containing solution was loaded onto the column, which had been previously equilibrated with 2 M (NH₄)₂SO₄, 0.15 M NaCl, 20 mM NaPO₄, pH 7.0. After sample loading, the column was washed with 2 M (NH₄)₂SO₄, 0.15 M NaCl, 20 mM NaPO₄, pH 7.0. The desired protein was then eluted using a decreasing linear (NH₄)₂SO₄ gradient ranging from 2-0 M developed in 0.15 M NaCl, 20 mM NaPO4, pH 7.0. When the desired protein began to elute, as indicated by an increase in the A₂₈₀ of the eluate, fractions were collected. Aliquots of each fraction were then analyzed by SDS-PAGE. Those fractions containing the desired protein were then pooled, thoroughly mixed, and the volume of the pool determined, as was the concentration of the protein therein.

The pooled HIC protein-containing eluate was then concentrated and the elution buffer exchanged. Typically, proteins were concentrated to 5.0-10.0 mg/mL. Ultrafiltration was conducted using an ultrafiltration system equipped with a PTGC Pellicon™ cassette system (Millipore, Inc., Bedford, MA) with an appropriately sized cut-off membrane

After concentration, the sample was diafiltered against an appropriate buffer. The retentate from the concentration step was diafiltered against 0.15 M NaCl, 20 mM NaPO₄, pH 7.0 until the conductivity of the retentate was within 5% of the conductivity of the 0.15 M NaCl, 20 mM NaPO₄, pH 7.0 solution.

In addition, to remove precipitates and bacterial endotoxin that might be present, the concentrated diafiltered protein-containing sample was passed through a 0.1 µm Posidyne™ filter (Pall, Inc., Cortland, NY). After determining the protein concentration of the solution and on the basis of the desired concentration of the final bulk product, the solution was diluted with 0.15 M NaCl, 20 mM sodium phosphate, pH 7.0, to the desired final concentration. A final aseptic filtration through a 0.22 µm filter was then performed as the final bulk product was transferred to a pyrogen-free container for storage (at about 5°C) for further formulation.

### B. Analysis

Analysis was conducted using *E. coli*-derived, native KGF; C(1,15)S; C(1,15)S/R(144)Q; ΔN15; ΔN23 and ΔN23/R(144)Q.

### Conformational Stability

The polypeptides were compared by their storage stability, thermal unfolding transition temperatures (Tm), and stability in a broad range of pH conditions.

### Storage Stability

Native KGF and C(1,15)S were incubated at 37°C for about 24 hrs, and the remaining soluble protein determined. The results are summarized in Figures 24 to 26.

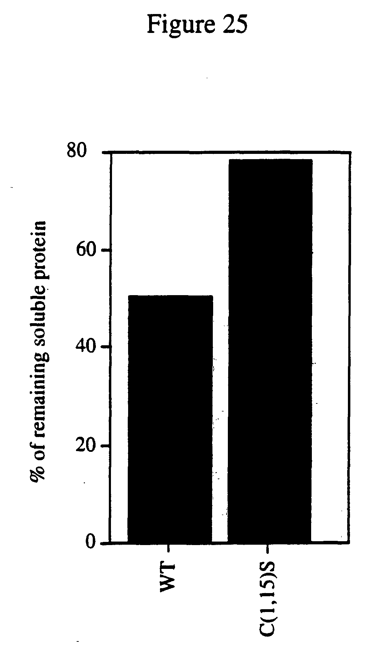

Figure 25 compares native KGF and C(1,15)S when stored in 20 mM NaPO₄ 0.15 M NaCl, pH 7.0 at 37°C for 27 hrs. C(1,15)S showed a significantly increased amount of soluble protein relative to native KGF.

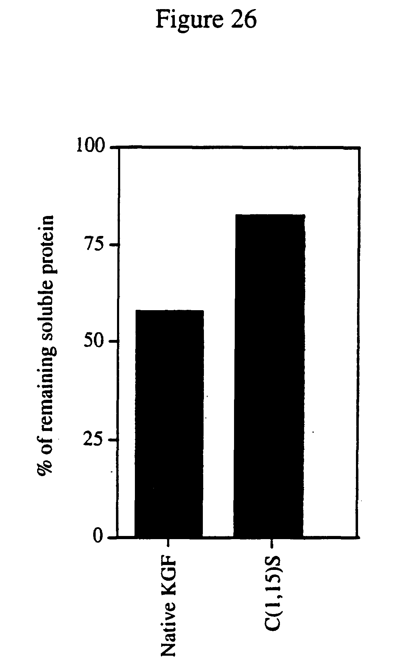

Figure 26 compares native KGF and C(1,15)S stored in 0.15 M NaCl, 20 mM NaPO₄, 0.15 M NaCl, pH 7.0 when stored at 37°C. Here again, C(1,15)S gave enhanced stability relative to native KGF.

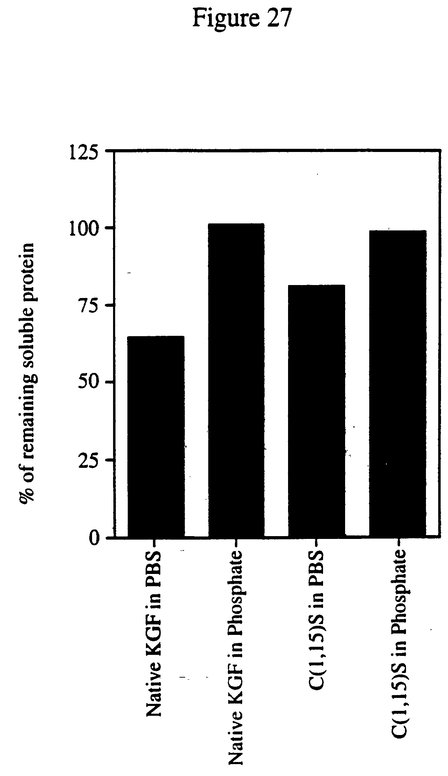

Figure 27 compares the stability of native KGF, ΔN15 and C(1,15)S in PBS and in 50 mM NaPO₄, 0.15 M NaCl, pH 7.0, when stored at 37°C for 18 hrs. The recovery of soluble protein is much higher in high phosphate than in PBS (100 vs. 65%). ΔN15 (data not shown) as well as C(1,15)S showed significantly enhanced stability, as compared with native KGF. ΔN15 and C(1,15)S also gave ^{~}100% recovery in high phosphate, as expected from the result of native KGF.

However, a single preliminary comparative example of storage stability was conducted between C(1,15,40)S, and C(40)S (which is encoded by bases 201 to 684 of SEQ ID NO:1, except that Ser⁴⁰ is encoded by AGA); and between C(1,15,102), and C(102)S (which is encoded by bases 201 to 684 of SEQ ID NO:1, except that Ser¹⁰² is encoded by AGG). The results (not shown) indicated a decreased stability (i.e., less soluble protein after being stored at 37°C). However, the amount of soluble, correctly folded C(1,15,40)S protein which was purified from the culture medium was greater than that of C(40)S, suggesting that C(1,15,40)S may in fact be more stable and that the results from the comparative example are inconclusive. The present invention preferably includes a KGF analog having other than substitutions at Cys⁴⁰, Cys¹⁰², Cys¹⁰⁶ and more preferably excludes C(1,15,40)S, C(1,15,102)S and C(1,15,40,102,106)

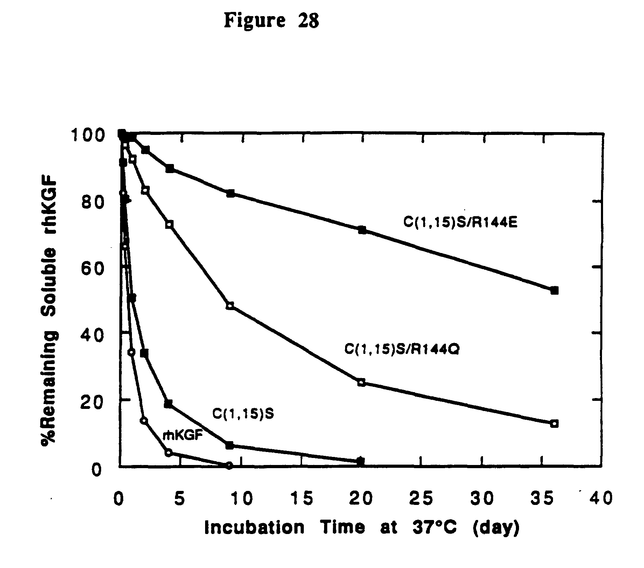

The ability of native KGF, C(1,15)S, ΔN23, C(1,15)S/R(144)E,C(1,15)S/R(144)Q and ΔN23/R(144)Q to prevent aggregation at elevated temperatures was also examined. Samples containing 0.5 mg/mL of protein were prepared in D-PBS. 0.5 mL of each sample was aliquoted into 3 cc type-1 glass vials. The vials were sealed with rubber stoppers and 13 mm flip-off aluminum seals were crimped on. These vials were then placed in a 37°C incubator. At predetermined time intervals, vials were withdrawn and analyzed for the loss of soluble protein. Visible precipitates were removed by centrifuging 250 µL of each sample through a 0.22 µm Spin-X™ filter unit (Costar, Cambridge, MA). Soluble protein in the filtered solutions was subsequently analyzed by size exclusion HPLC. The amount of soluble protein was determined by integrating the HPLC peak area and plotting the result as a function of incubation time at 37°C. The results for native KGF, C(1,15)S, C(1,15)S/R(144)E, and C(1,15)S/R(144)Q are shown in Figure 28. The data for ΔN23 and ΔN23/R(144)Q are not shown.

The half-lives for the loss of soluble, monomeric protein were then estimated from these kinetic curves. Table 1 shows the half-life for remaining soluble KGF upon storage at 37°C for these proteins.

**Table 1**

| Half-life for the Loss of Soluble, Monomeric Proteins | |
|---|---|
| Protein | t1/2 (day) |
| Native KGF | 0.6 |
| ΔN23 | 1.1 |
| C(1,15)S | 1.2 |
| C(1,15)S/R(144)Q | 13.3 |
| ΔN23/R144Q | 22.3 |
| C(1,15)S/R(144)E | 38.0 |

As seen in Table 1, above, and Figure 28, the native KGF aggregated the most rapidly, with a solubility half-life of 0.6 days. C(1,15)S/R(144)Q, ΔN23/R(144)Q and C(1,15)S/R(144)E showed substantial increases in the solubility half-life to 13.3, 22.3 and 38 days, respectively.

### Thermal Unfolding

Thermal unfolding was monitored by circular dichroism (CD) at 230 nm using a J-720™ spectropolarimeter (Jasco, Inc., Easton, MD) equipped with a PTC-343 Peltier-type temperature control system. For CD analysis, separate samples containing 0.1 mg/mL of the polypeptide to be analyzed were prepared in D-PBS (Life Technologies, Inc., Grand Island, NY). For each sample, about 2.5 mL was loaded into a 10 mm path length rectangular Suprasil™ quartz (Heraeus Quarzschmelze, GmbH, Hanau, Germany) fluorescent cell (Hellma Cells, Inc., Jamaica, NY). The cell was then placed into the Peltier-type temperature control system in the spectropolarimeter. Thermal unfolding was carried out at a rate of 50°C/hr. Changes in ellipticity were monitored at 230 nm to indicate unfolding. The Tₘ of each sample was estimated by identifying a temperature at which 50% of protein molecules in the solution were unfolded (*Biophysical Chemistry,* Cantor and Schimmel (eds), W.H. Freeman and Co. San Francisco (1980). The estimated Tₘ for each of the three proteins is listed in Table 2.

**Table 2**

| Estimated Melting Temperatures | |
|---|---|
| Protein | Tₘ (°C) |
| native KGF | 54.0 |
| ΔN15 | 55.0 |
| C(1,15)S | 55.0 |
| ΔN23 | 56.0 |
| C(1,15)S/R(144)Q | 62.5 |
| ΔN23/R144Q | 63.0 |
| C(1,15)S/R(144)E | 63.5 |

As these results show, C(1,15)S and ΔN15 have a 1°C increase in the Tₘ as compared with native KGF. The ΔN23 has an additional degree increase in Tm. However, the substitution of R144Q to C(1,15)S/R(144)Q or ΔN23 adds a greater than 6°C increase in Tₘ and more than 7°C as compared with native KGF. Moreover, C(1,15)S/R(144)E is greater than 9°C more stable than native KGF.

### pH

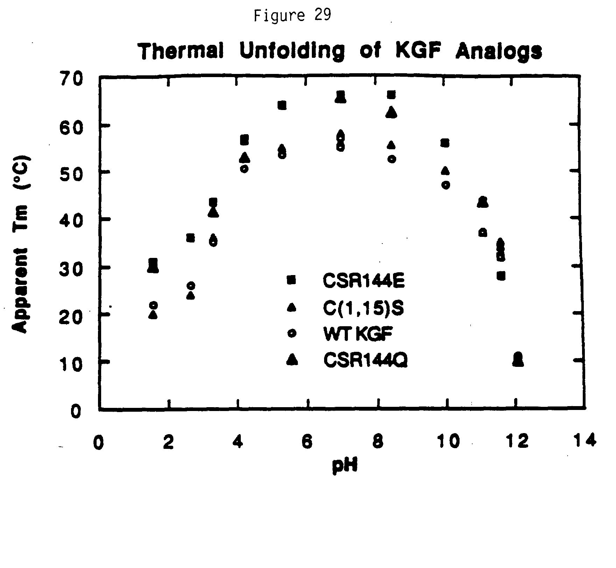

The acid stabilities of C(1,15)S/R(144)Q and C(1,15)S/R(144)E were also compared to that of native KGF, by adjusting D-PBS to different pH values by adding concentrated HCl or NaOH. Approximately 2.35 mL of D-PBS at different pH values was mixed with 100 µL of 2.45 mg/mL KGF protein in a quartz cell. These samples were thermally unfolded at a rate of 50°C/hr and monitored by CD at 230 nm. Figure 29 shows the Tₘ as a function of pH for native KGF, C(1,15)S, C(1,15)S/R(144)Q and C(1,15)S/R(144)E. In the pH range tested, the C(1,15)S,C(1,15)S/R(144)Q and C(1,15)S/R(144)E always have a higher Tₘ than the native KGF.

### In vitro Biolocrical Activity

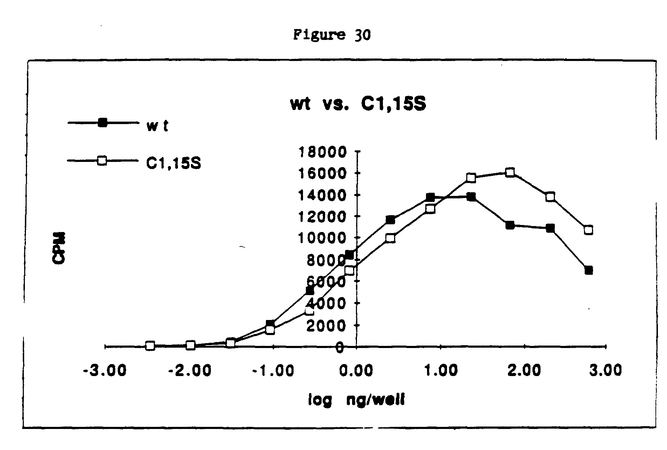

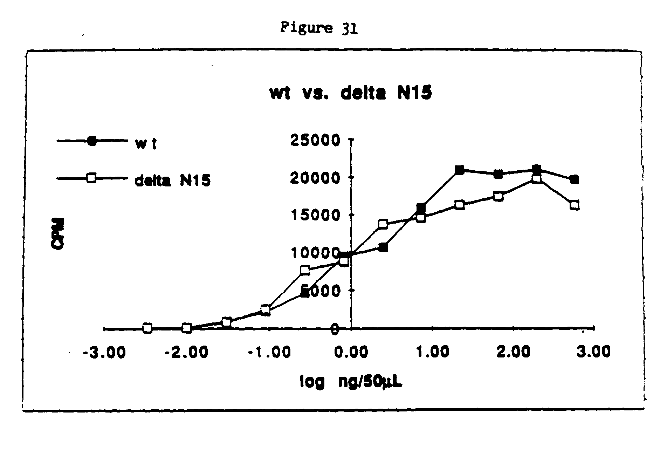

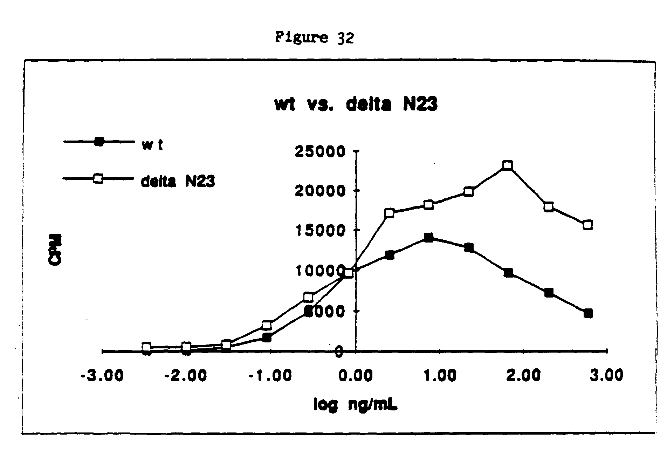

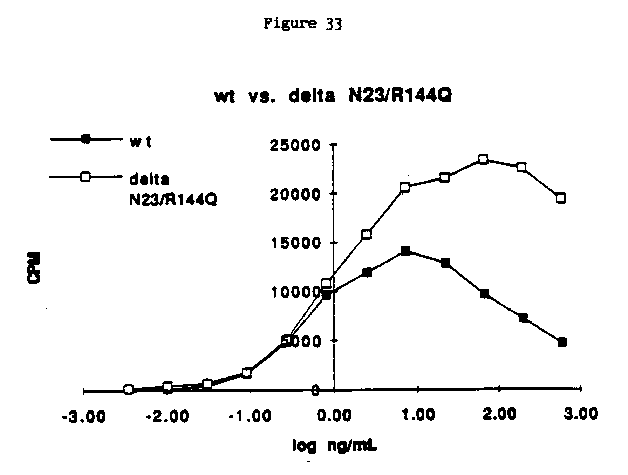

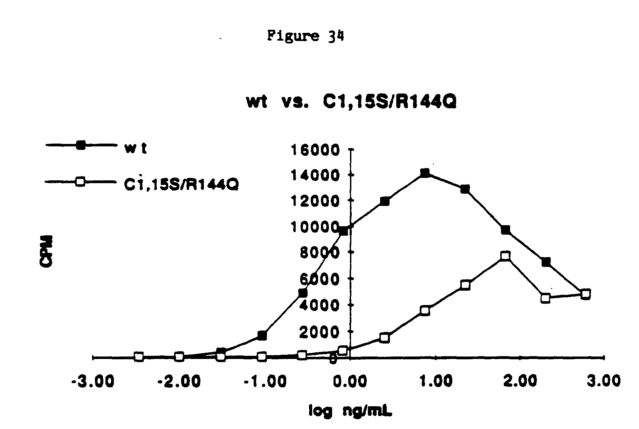

*In vitro* mitogenic activity of C(1,15)S, ΔN15, ΔN23, ΔN23/R(144)Q, C(1,15)S/R(144)Q and C(1,15)S/R(144)E was also determined as a function of protein concentration and the half-maximal concentrations by measurement of [³H]-thymidine uptake by Balb/MK cells (according to the methods of Rubin *et al*. (1989), *supra).* Generally, the concentrations of each of the KGF analogs relative to a known standard native KGF was determined using an *in vitro* biological assay. Each KGF analog was then diluted and assayed for biological activity using a Balb/MK mitogenic assay. The samples were first diluted in a bioassay medium consisting of 50% customer-made Eagle's MEM, 50% customer-made F12, 5 µg/ml transferrin, 5 ng/ml sodium selenite, 0.0005% HSA and 0.005% Tween 20. KGF samples were then added into Falcon Primeria 96-well plates seeded with Balb/MK cells. Incorporation of [³H]-Thymidine during DNA synthesis was measured and converted to input native KGF concentration by comparison to a native KGF standard curve. The results are presented in Figures 30 to 35. As seen in the Figures, the tested KGF analogs described in Figures 29 to 34 have comparable activity to native KGF.

### Example 3: Production in Mammalian Cell Culture

This example describes the expression, isolation, and characterization of two biologically active recombinant KGF (rKGF) forms produced in a mammalian expression system.

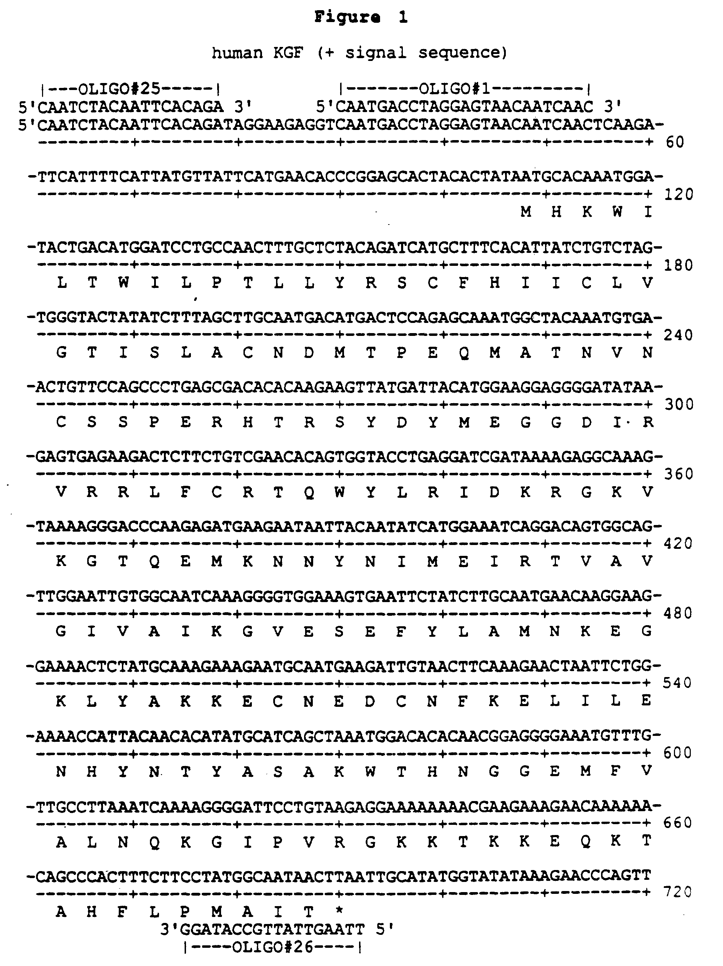

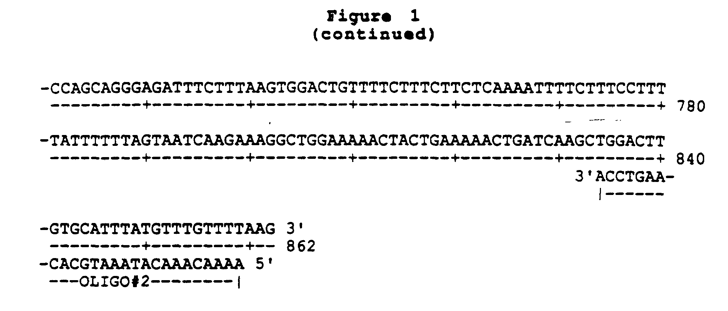

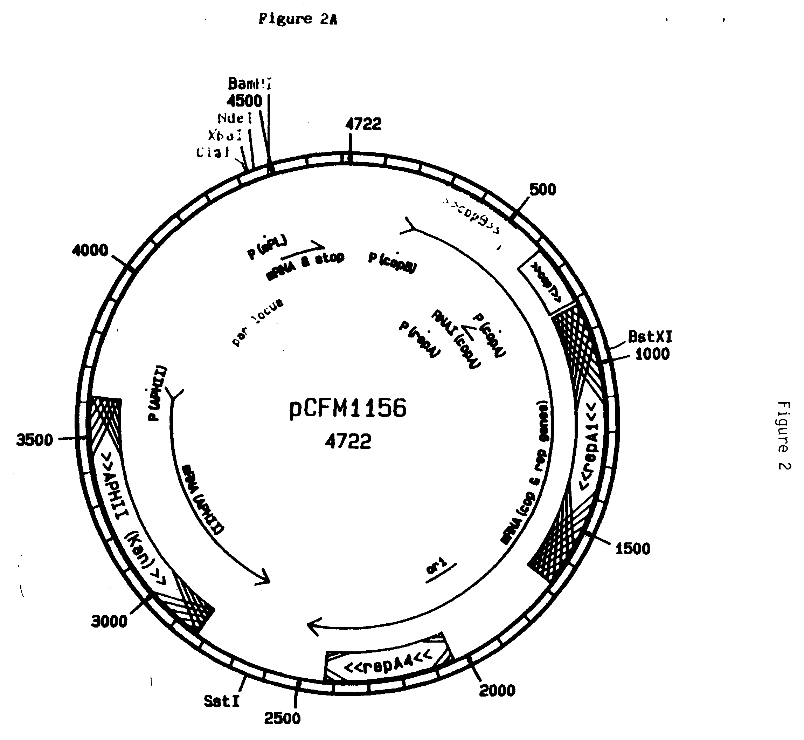

The human KGF gene was isolated by PCR amplification of cDNA made from normal dermal human fibroblast cells (Clonetec, Inc., San Diego, CA). Following the making of cDNA by reverse transcriptase, PCR was used to amplify the KGF gene. OLIGO#25 and OLIGO#26 were used to amplify the gene out of the cDNA, and OLIGO#27 and OLIGO#28 were used to place *Hind*III and *Bgl*II restriction sites at the fragment ends by a second PCR amplification, as set forth in Figure 1.

OLIGO#25 (SEQ ID NO:69): 5'-CAATCTACAATTCACAGA-3'
OLIGO#26 (SEQ ID NO:70): 5'-TTAAGTTATTGCCATAGG-3'
OLIGO#27 (SEQ ID NO:71): 5'-AACAAAGCTTCTACAATTCACAGATAGGA-3'
OLIGO#28 (SEQ ID NO:72): 5'-AACAAGATCTTAAGTTATTGCCATAGG-3'

Following cloning and DNA sequence confirmation, the KGF gene DNA was then used. Amplification was effected using OLIGO#29 and OLIGO#30.

OLIGO#29 (SEQ ID NO:73):
5'-CGGTCTAGACCACCATGCACAAATGGATACTGACATGG-3'
OLIGO#30 (SEQ ID NO:74):
5'-GCCGTCGACCTATTAAGTTATTGCCATAGGAAG-3'

The sense primer, OLIGO#29, included an *Xba*I site and a consensus Kozak translation sequence (5'-CCACC-3') upstream of the start codon, ATG. The antisense primer, OLIGO#30, included a *Sal*I cloning site and an additional stop codon. After 18 cycles of PCR amplification (30 sec. denaturation at 94°C, 40 sec. annealing at 55°C, and 40 sec. elongation at 72°C), the product was digested with *Xba*I and *SalI* and ligated with a similarly digested DNA of pDSRα2 (according to the methods of Bourdrel *et al.* (1993), *Protein Exp. & Purif.,* 4:130-140 and Lu *et al.* (1992), *Arch. Biochem. Biophys*., 298:150-158). This resulted in plasmid KGF/pDSRα2 which placed the human KGF gene between the SV40 early promoter and the α-FSH polyadenylation sequences. Two clones were picked and DNA sequence analysis confirmed construction of the desired vector.

Two micrograms of KGF/pDSRa2 DNA were then linearized with *Pvu*I. Chinese hamster ovary (CHO) cells, seeded the day before at 0.8 x 10⁶ cells/60 mm culture dish, were then transfected with the treated DNA using a standard calcium phosphate precipitation method (Bourdrel *et al., supra).* Two weeks later, individual colonies were picked and transferred into 24-well plates. The conditioned media was considered serum free when the cells reached confluency and aliquots thereof were analyzed by Western blotting using a polyclonal rabbit antiserum reactive against E. coli-expressed human KGF.

Westerns were performed by running samples through 12.5% (w/v) SDS polyacrylamide gels, followed by electroblotting for 1 hr. at 400 mA onto nitrocellulose membranes using a semidry transfer apparatus (Hoefer Scientific Instruments, San Francisco, CA). 20 mM Tris, 150 mM glycine, 20% methanol served as the transfer buffer. The nitrocellulose sheets were blocked by incubation with 10% normal goat serum in PBS. Rabbit anti-serum raised against *E. coli*-derived KGF was used as primary antibody. For use, it was diluted 1/10,000 in 1% normal goat serum in PBS and incubated with the blocked nitrocellulose sheets for 12 hr. at room temperature, after which excess antibody was removed by three 30 min. washes in PBS. The nitrocellulose membranes were then incubated in 100 mL of 1% normal goat serum in PBS containing Vectastain™ biotinylated goat anti-rabbit IgG (secondary antibody, Vector Labs, Burlingame, CA) for 30 minutes at room temperature. After three 10-minute washes in PBS, a 30-minute room temperature incubation was performed in a 100 mL solution of 1% normal goat serum containing streptavidin and biotinylated peroxidase, prepared according to manufacturer's directions (Vector Labs). Following three washes in PBS, KGF cross-reactive material was visualized by incubation in a mixture of 60 µL of 30% (w/v) H₂O₂ in 100 mL of PBS and 50 mg of 4 chloronapthol in 20 mL of methanol. The reaction was stopped by rinsing in water after 10 minutes.

Analysis of the blots revealed that the KGF-specific antibody associated with three distinct protein bands, two being closely related with molecular weights of about 25-29 kDa and one with an estimated molecular weight of about 17 kDa, as compared to the expected molecular weight of approximately 18.8 of the 163 amino acid mature protein. Additionally, several high-expressing clones secreting more than 2.0 mg of rKGF per liter, as judged by Western analysis, were selected and expanded into roller bottles (according to the method of Lu *et al., supra)* to generate large volumes of serum-free conditioned medium for purification of KGF by cationic exchange chromatography and gel filtration, as set forth below.

KGF from 3 L of serum-free conditioned medium was purified applying the medium directly to a cation exchange column (5 x 24 cm) packed with 450 mL of sulfoethyl a column of S-Sepharose Fast Flow (Pharmacia) pre-equilibrated with 20 mM sodium phosphate, pH 7.5. After washing with five column volumes of 20 mM sodium phosphate, 0.2 M NaCl, pH 7.5, rKGF was eluted using a 20-column volume linear gradient of 0.2 to 1.0 M NaCl in 20 mM sodium phosphate, pH 7.5. 50 mL fractions were collected with continuous A₂₈₀ monitoring. KGF protein was detected by analyzing aliquots of each fraction by SDS-PAGE. SDS-PAGE was performed on an electrophoresis system (Novex, San Diego, CA) using precast 14% Tris-glycine precast gels (according to the method of Laemmli (1970) *Nature,* 227:680-685). Samples were mixed with non-reducing SDS sample buffer without heating before loading. The proteins were detected by either Coomassie blue or silver staining. Two late-eluting peaks were seen to contain protein bands corresponding to the 25-29 kDa and--17 kDa bands detected by Western blot. The fractions containing each of these peaks were separately concentrated to a volume of less than 1.0 mL and subjected to gel filtration.

The gel filtrations employed columns of Superdex-75™ resin (HR 10/30, Pharmacia) pre-equilibrated with PBS, pH 7.2, and calibrated with the following known molecular weight-standards (BioRad, San Francisco, CA): thyroglobulin (670 kDa), g-globulin (158 kDa), ovalbumin (44 kDa), myoglobin (17 kDa) and vitamin B-12 (1.4 kDa). These purification steps resulted in an approximate 2000-fold purification of rKGF, specifically including a 17 kDa and a 30 kDa material, as estimated by silver staining.

In the instance of the higher molecular weight material, rKGF eluted as a major symmetrical peak (by A₂₈₀) which was called KGF-a. Upon SDS-PAGE analysis of a lesser amount of this material, 3 µg/lane versus 6 µg/lane, two bands with a 1-2 kDa molecular weight difference were resolved. In the instance of the lower molecular weight material, termed KGF-b, gel filtration resulted in a protein preparation having the expected mobility. For both KGF-a and KGF-b, the overall yield after purification was approximately 30-40%.

Amino acid sequences from KGF-a and KGF-b were also analyzed. These analyses were performed on an automatic sequencer (Model 477A or 470A, Applied Biosystems, Inc., Foster City, CA) equipped with a Model 120A on-line PTH-amino acid analyzer and a Model 900A data collection system (according to the method of Lu *et al.* (1991), *J. Biol. Chem*., 266:8102-8107). Edman sequence analysis of KGF-a revealed a major N-terminal sequence of X₁-N-D-M-T-P-E-Q-M-A-T-N-V-X₂-X₃-S- [SEQ ID NO:75]. A minor sequence starting from the third N-terminal amino acid, aspartic acid, was also present in 1.6% of the total sequenceable protein. X₁, X₂, and X3 were the unassigned due to the absence of phenylthiohydantoinyl (PHT) amino acid signals during sequence analysis. An N-terminal amino acid sequence of KGF predicted from cDNA sequence indicates that X₁ and X₃ are Cys residues and X₂ is asparagine. The absence of X₁ and X₃ indicates that these cysteines may form disulfide bridges. On the basis of the consensus N-linked glycosylation sequence Asn-X-Ser, the absence of the predicted Asn residue corresponding to X₂ indicates that it is a potential N-linked glycosylation site.

Interestingly, N-terminal sequence analysis of KGF-b revealed an N-terminal amino acid sequence of S-Y-D-Y-M-E-G-G-D-I-R-V- (SEQ ID NO:76), indicating that it is an N-terminally truncated form of KGF that has been proteolytically cleaved at the Arg²³-Ser²⁴ peptide bond.

To further characterize purified KGF-a and KGF-b, the protein was subjected to glycosidases (neuraminidase, O-glycanase, and/or N-glycanase), using known techniques (Sasaki *et al.* (1987), *J. Biol. Chem.,* 262:12059-12076; Takeuchi *et al.* (1988), *J. Biol. Chem*., 263:3657-3663; Zsebo *et al.* (1990), *Cell,* 63:195-201). These data indicate that KGF-a contains N- and O-linked carbohydrates, although the lower molecular weight form of KGF-a probably contains only N-linked sugar. Glycosidase treatment did not cause molecular weight reduction for KGF-b, indicating that the molecule is unglycosylated.

The glycosylation pattern of KGF-a was further characterized by mass spectroscopy of the endoproteinase Glu-C-generated peptides described above. Elucidation of carbohydrate structure of glycopeptides by the stepped orifice method of mass spectrometric analysis has been successfully applied to other proteins (Huddleston *et al.* (1993), *Anal. Chem.,* 65:877-884; Carr *et al.* (1993), *Prot. Sci.,* 2:183-196). As confirmed by the isolation of a non-glycosylated peptide, Thr²² seems to be partially glycosylated. Similar mass spectrometric analysis of Asn¹⁴ suggested microheterogeneity in glycosylation, with bi, tri and tetra-antennary structures with varying degrees of sialylation.

Table 3A summarizes the KGF concentration to stimulate [³H]-thymidine incorporation of keratinocytes at half-maximal rate (according to the method of Rubin et al. (1989), *Supra*). Interaction with the KGF receptor was examined using isolated KGF receptor membrane preparations prepared from Balb/MK mouse epidermal keratinocytes (by the procedure described by Massague (1983), *J. Biol. Chem.,* 258:13614-13620). Specifically, various forms of KGF were diluted with 50 mM Tris-HCl, pH 7.5, containing 0.2% bovine serum albumin so as to range in concentration from 0.8 ng to 100 ng per 50 µL. They were individually incubated with the membrane preparation (75 ng/mL) and ¹²⁵I-labeled E. coil-derived KGF (1.5 ng). Receptor binding and competition experiments were performed at 4°C for 16 hr., after which time samples were taken, centrifuged, and washed twice with the above diluent buffer to remove unbound and non-specifically bound, labeled KGF. Samples were then counted for the remaining radioactivity. Competition curves for receptor binding between KGF samples and labeled KGF were constructed by plotting percent uncompetition versus concentrations of each KGF sample. Table 3B summarizes the KGF concentration needed to achieve 60% uncompetition against the labeled *E. coli* derived KGF, expressed as ng/mL.

**Table 3A**

| KGF concentration to stimulate [³H]-thymidine incorporation of keratinocytes at half-maximal rate | |
|---|---|
| Forms | ng/mL |
| *E. coli* KGF | 10 |
| KGF-a | 30 |
| KGF-b | 30 |

**Table 3B**

| KGF concentration to compete receptor binding with I¹²⁵-labeled KGF at 60% uncompetition rate | |
|---|---|
| Forms | ng/mL |
| *E. coli* KGF | 65.8 |
| KGF-a | 93.5 |
| KGF-b | 89.1 |

As shown in Table 3A, KGF-a and KGF-b stimulate comparable [³H]-thymidine incorporation, with the half-maximal rate being stimulated by approximately 30 ng/ml of either analog. Thus, the truncation does not reduce biological activity of the molecule. However, the two analogs have approximately 3-fold lower activity than the *E. coli*-derived, full-length KGF. As shown in Table 3B, radioreceptor assay uncompetition experiments indicated that *E. coli*-derived KGF, KGF-a, and KGF-b have similar receptor binding activity.

### Example 4: In Vivo Biological Assay of KGF Polypeptides Produced in E. coli and Mammalian Cell Culture

A single subcutaneous dose of KGF has been shown to result in a dose-dependent rise in serum cholesterol in mice within 24 hours. Native KGF, KGF-a, C(1,15)S, ΔN15 and ΔN23 were also evaluated for the ability to raise the serum cholesterol level in a dose-dependent manner.

Each treatment group contained five female Balb/c mice (18-20 gms) obtained from CRL. The protein was diluted with 0.9% saline to achieve a final injection volume of 100 µl/mouse. Each mouse was administered a single subcutaneous injection, at the following doses:

| **Group** | **Treatment** | **Dose (mg/kg)** |
|---|---|---|
| 1 | Native KGF | 0.1 |
| | Native KGF | 0.25 |
| | Native KGF | 0.5 |
| | Native KGF | 1 |
| | Native KGF | 5 |
| | | |
| 2 | C(1,15)S | 0.1 |
| | C(1,15)S | 0.25 |
| | C(1,15)S | 0.5 |
| | C(1,15)S | 1 |
| | C(1,15)S | 5 |
| | | |
| 3 | ΔN15 | 0.25 |
| | ΔN15 | 0.5 |
| | ΔN15 | 1 |
| | ΔN15 | 5 |
| | | |
| 4 | ΔN23 | 0.1 |
| | ΔN23 | 0.5 |
| | ΔN23 | 1 |
| | ΔN23 | 5 |
| | | |
| 5 | KGF-a | 0.01 |
| | KGF-a | 0.05 |
| | KGF-a | 0.1 |
| | KGF-a | 0.5 |
| | | |
| 6 | Saline Control | - |

Twenty-four hours after injection, the mice were sacrificed and bled via cardiac puncture. Blood samples were processed for the determination of serum cholesterol.

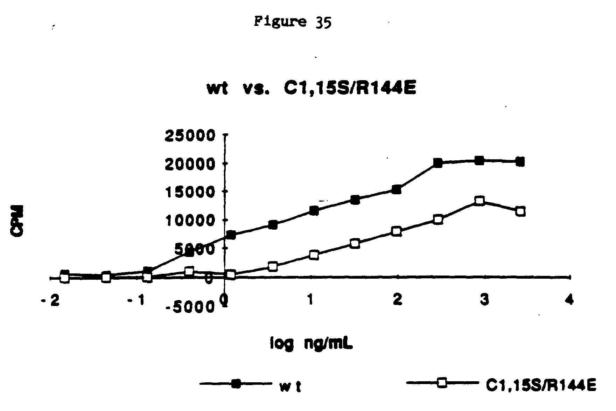

As shown in Figure 35, each tested KGF analog was found to raise serum cholesterol in a dose dependent manner. Also, there was no apparent difference in bioactivity between any of the tested KGF analogs.

### In Vivo Model of Diabetes

Chemically-induced diabetes mellitus models in various animal species have been classically used to study the disease and its treatment. Streptozotocin induces diabetes in the mouse, rat, hamster, dog, and monkey although studies in rats and mice are utilized most. Junod *et al., Proc. Soc. Exp. Pio. Med.* **126:**210-205 (1967); Rerup, *Pharm. Rev.* **22:**485-518 (1970); Rossini *et al.,* P.N.A.S. **74:**2485-2489 (1977); and *Ar'Rajab and Ahren, Pancreas* **8:**50-57 (1993). In rats, doses of streptozotocin from 45 to 70 mg/kg as a single intravenous dose induce stable disease. Doses below 45 mg/kg induce a transient disease state which is reversible. Within one day of streptozotocin injection, the hyperglycemic state is induced. Blood insulin levels remain essentially unchanged compared with normal rats; however, the total content of insulin and C-peptide in the pancreas is severely decreased. Rats manifest the classic signs and symptoms of diabetes in humans:
increased blood glucose levels (hyperglycemia), glucose in the urine (glucosuria), increased thirst (polydipsia), increased urination (polyuria), increased appetite (hyperphagia).

The studies described in this disclosure were carried out with the streptozotocin-induced diabetes model in Sprague-Dawley rats. Male rats weighing 200 260 grams at study initiation were used. Diabetes was induced by a single intravenous injection of streptozotocin at 50 mg of streptozotocin in sodium citrate buffer per kg of body weight. Non-diabetic control rats received a single intravenous injection of sodium citrate buffer for control purposes. KGF was administered daily as a subcutaneous injection. The KGF dose was 3 or 5 mg/kg/day, depending upon the experiment. In the first experiment, KGF therapy was initiated two days before diabetes, was induced and continued after the induction of diabetes for a total of eight injections. In the second and third experiments, KGF therapy administered subcutaneously was initiated one day after the induction of diabetes with streptozotocin. In the fourth experiment, a 7 day course of KGF therapy was initiated 7 days after streptozotocin treatment and the animals were then followed for an additional 12 weeks. In all experiments, except for the fourth experiment, blood glucose levels, urine glucose levels and urine volume were used as end points for analysis. Additionally, water intake, urine C-peptide levels, or total pancreatic insulin and C-peptide content were measured in some experiments. In the fourth experiment, the only assessed endpoint was blood glucose. Because a large fraction of insulin is removed from the circulation by the liver, measurement of peripheral insulin concentrations reflect post-hepatic metabolism events rather than insulin secretion from the pancreas. Therefore, measurements of C-peptide are often made and used as a peripheral marker of insulin secretion. C-peptide is produced from the processing of pro-insulin to insulin. Insulin and C-peptide are secreted from the beta cells in equimolar amounts, and only a small amount of C-peptide is extracted by the liver.

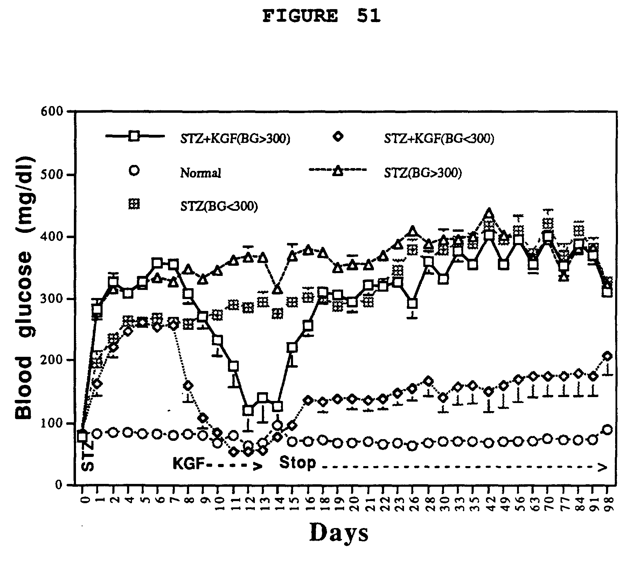

This study investigated the effect of rKGF on streptozotocin-induced diabetes in Sprague-Dawley rats. On day 0, groups of rats were exposed to either 45 or 50 mg/kg streptozotocin (STZ). Following these treatments, non-fasting blood glucose levels were monitored daily to assess the severity of the islet injury. On day 5, the STZ-treated animals were placed into one of two groups (20/group) depending on the magnitude of hyperglycemia. The dividing point was set at a blood glucose level of 300 mg/dl. A group of non STZ-treated animals served as controls. On day 7, 10 animals from each hyperglycemic group were given ΔN23 (3 mg/kg/day) or PBS by subcutaneous injection for 7 days. Blood glucose levels were then monitored daily, every other day, or weekly and are set forth in Figure 51. Note that STZ-treated animals from both groups receiving ΔN23 had significant declines in blood glucose during the ΔN23 dosing period. Importantly, the mean blood glucose drop experienced by the STZ-treated animals from the <300 mg/dl starting blood glucose group stabilized at about 150 mg/dl whereas the blood glucose drop seen in the >300 mg/dl starting blood glucose group was only transient. Note that the day scale is non-linear.

## Claims

1. A polypeptide analog of native keratinocyte growth factor termed "KGF," wherein native KGF corresponds to the following sequence 
Cys Asn Asp Met Thr Pro Glu Gln Met Ala Thr Asn Val Asn Cys Ser Ser
1               5                   10                  15

Pro Glu Arg His Thr Arg Ser Tyr Asp Tyr Met Glu Gly Gly Asp Ile
        20                  25                  30

Arg Val Arg Arg Leu Phe Cys Arg Thr Gln Trp Tyr Leu Arg Ile Asp
    35                  40                  45

Lys Arg Gly Lys Val Lys Gly Thr Gln Glu Met Lys Asn Asn Tyr Asn
50                  55                  60                  65

Ile Met Glu Ile Arg Thr Val Ala Val Gly Ile Val Ala Ile Lys Gly
                70                  75                  80

Val Glu Ser Glu Phe Tyr Leu Ala Met Asn Lys Glu Gly Lys Leu Tyr
            85                  90                  95

Ala Lys Lys Glu Cys Asn Glu Asp Cys Asn Phe Lys Glu Leu Ile Leu
        100                 105                 110

Glu Asn His Tyr Asn Thr Tyr Ala Ser Ala Lys Trp Thr His Asn Gly
    115                 120                 125

Gly Glu Met Phe Val Ala Leu Asn Gln Lys Gly Ile Pro Val Arg Gly
130                 135                 140                 145

Lys Lys Thy Lys Lys Glu Gln Lys Thr Ala His Phe Leu Pro Met Ala
150 155 160

Ile Thr,
 with or without a signal sequence, wherein the analog comprises the amino acid sequence of KGF comprising a deletion, substitution and/or insertion of one or more of amino acids 1-24 whereby the cysteine residue at amino acid position 1 and the cysteine residue at amino acid position 15 are each either deleted or substituted with another amino acid optionally further having an N-terminal methionine residue and/or further comprising a charge-change substitution at an amino acid position selected from Arg (41), Glu (43), Lys (55), Lys (95), Asn (137), Gln (138), Lys (139), Arg (144), Lys (147), Gln (152), Lys (153) or Thr (154), or in case of Δ23-KGF having a charge-change modification of His (116), or in case of native KGF having both Cys (1) and Cys (15) substituted by Ser optionally having also Cys (40) or Cys (102) or both Cys (102) and Cys (106) substituted by Ser with the proviso that the analog is not the keratinocyte growth factor protein consisting of amino acids 24-163.

2. The analog according to claim 1, wherein the cysteine residue at amino acid position 1 and the cysteine residue at amino acid position 15 are deleted.

3. The analog according to claim 1, wherein amino acids 1-24 are deleted.

4. The analog according to claim 1, wherein the cysteine residue at amino acid position 1 is deleted and the cysteine residue at amino acid position 15 is substituted with another amino acid.

5. The analog according to claim 1, wherein at least one amino acid of amino acids 1 to 24 is deleted and the cysteine residue at amino acid position 15 is substituted with another amino acid.

6. The analog according to claim 1, wherein the cysteine residue at amino acid position 1 and the cysteine residue at amino acid position 15 are substituted with another amino acid.

7. The analog according to any one of Claims 4 to 6, wherein an amino acid residue selected from alanine, leucine or serine is substituted for a cysteine residue.

8. The analog according to Claim 7, wherein a serine residue is substituted for a cysteine residue.

9. The analog according to any one of claims 1 to 8 comprising a charge-change substitution at an amino acid position selected from the arginine residue at amino acid position 41, the glutamine residue at amino acid position 43, the lysine residue at amino acid position 55, the lysine residue at amino acid position 95, the asparagine residue at amino acid position 137, the glutamine residue at amino acid position 138, the lysine residue at amino acid position 139 the arginine residue at amino acid position 144, the lysine residue at amino acid position 147, the glutamine residue at amino acid position 152, the lysine residue at amino acid position 153, or the threonine residue at amino acid position 154.

10. The analog according to any one of claims 1 to 9 comprising a charge-change modification of the histidine residue at amino acid position 116.

11. The analog according to claim 10, wherein the histidine residue is replaced by a glycine residue.

12. The analog according to any one of claims 9 to 11, wherein amino acids 1-24 are deleted.

13. The analog according to claim 1, wherein the analog is C(1,15)S; C(1,15,40)S; C(1,15,102)S; C(1,15,102,106)S; ΔN15; ΔN16; ΔN17; ΔN18; ΔN19; ΔN20; ΔN21; ΔN22; ΔN24; ΔN3/C(15)S; ΔN3/C(15)-; ΔN8/C(15)S; ΔN8/C(15)- and ΔN23/H(116)G which corresponds to amino acids 23-163, with the histidine residue at amino acid position 116 being substituted with a glycine.

14. The analog according to claim 13, wherein said analog is ΔN16.

15. The analog according to any one of claim 1 to 14, wherein said analog has a signal sequence or an amino-terminal methionine residue.

16. The analog according to claim 15, wherein said signal sequence is amino acids 1-31 of Fig. 1.

17. The analog according to any one of claims 1-6 wherein said analog is covalently attached to polyethylene glycol or related water soluble organic polymers.

18. The analog according to claim 17, wherein the analog is attached to polyethylene glycol.

19. An analog according to claim 1, wherein said analog is ΔN23 covalently attached to polyethylene glycol or related water soluble organic polymers.

20. The analog according to claim 19, wherein the analog is attached to polyethylene glycol.

21. The analog according to any one of claims 1 to 20, wherein said analog is lyophilized.

22. A pharmaceutical formulation comprising a therapeutically effective amount of an analog of KGF according to any one of claims 1 to 21 and a pharmaceutically acceptable carrier.

23. A recombinant nucleic acid molecule encoding an analog according to any one of claims 1 to 16.

24. A biologically functional vector comprising a nucleic acid molecule according to claim 23.

25. A procaryotic or eucaryotic host cell containing a nucleic acid molecule according to claim 23 or a biologically functional vector according to claim 24.

26. A procaryotic host cell according to claim 25 that is *E. coli.*

27. A eucaryotic host cell according to claim 25 that is a mammalian cell, preferably a Chinese hamster ovary cell.

28. A process for the production of an analog of KGF, the process comprising growing under suitable nutrient conditions a host cell according to any one of claims 25 to 27 in a manner allowing expression of the encoded analog, and isolating the analog so produced.

29. A use of an effective amount of the analog of KGF according to any one of claims 1 to 21 for the production of a medicament for stimulating production of non-fibroblast epithelial cells.

30. The use according to claim 29, wherein said non-fibroblast epithelial cells are selected from adnexal structures, liver cells, mucosal epithelium in the respiratory and gastrointestinal tracts, corneal cells or tympanic epithelial cells.

31. A use of an effective amount of the analog of KGF according to any one of claims 1 to 21 for the production of a medicament for stimulating production of non-fibroblast epithelial cells in a patient for the prevention or treatment of a condition, wherein said condition is selected from bums and other partial and full-thickness injuries; epidermolysis bullosa; chemotherapy-induced alopecia; male pattern baldness; progressive loss of hair in men and women; gastric and duodenal ulcers; inflammatory bowel diseases such as Crohn's disease and ulcerative colitis; gut toxicity in radiation and chemotherapy treatment regimes; hyaline membrane disease; acute or chronic lung damage; hepatic cirrhosis; fulminant liver failure; acute viral hepatitis; toxic insults to the liver; corneal abrasion; progressive gum disease or ear drum damage.

32. An *in vitro* method of stimulating the production of non-fibroblast epithelial cells comprising the administration of an effective amount of an analog of KGF, according to any one of claims 1 to 21, or a pharmaceutical composition of claim 22.

33. The use of Claim 29, wherein the non-fibroblast epithelial cells are stimulated in a patient in need thereof.

34. The method of Claim 32, wherein said non-fibroblast epithelial cells are selected from adnexal structures, liver cells, mucosal epithelium in the respiratory and gastrointestinal tracts, corneal cells or tympanic epithelial cells.

35. Use of an effective amount of ΔN23 for the production of a medicament for stimulating production of non-fibroblast epithelial cells in a patient in need thereof, wherein the non-fibroblast epithelial cells are selected from adnexal structures, liver cells, mucosal epithelium in the respiratory tract or tympanic epithelial cells.

36. An *in vitro* method of stimulating the production of non-fibroblast epithelial cells comprising the administration of an effective amount of ΔN23 or a pharmaceutical composition thereof, wherein the non-fibroblast epithelial cells are selected from adnexal structures, liver cells, mucosal epithelium in the respiratory tract or tympanic epithelial cells.

37. Use of an effective amount of ΔN23 for the production of a medicament for stimulating production of non-fibroblast epithelial cells in a patient for the prevention or treatment of a condition, wherein said condition is selected from burns and other partial and full-thickness injuries; epidermolysis bullosa; chemotherapy-induced alopecia; male pattern baldness; progressive loss of hair in men and women; gastric and duodenal ulcers; inflammatory bowel diseases such as Crohn's disease and ulcerative colitis; gut toxicity in radiation and chemotherapy treatment regimes; hyaline membrane disease; acute or chronic lung damage; hepatic cirrhosis; fulminant liver failure; acute viral hepatitis; toxic insults to the liver; progressive gum disease or ear drum damage.

38. The use according to any one of Claims 29,30,31,33,35 and 37, wherein said analog of KGF is administered via IV, IT, IM, SC, or IP routes.

39. A kit comprising an analog of KGF according to any one of Claims 1 to 21, or a pharmaceutical formulation of Claim 22.

## Patentansprüche

1. Ein Polypeptidanalogon des als "KGF" bezeichneten nativen Keratinocytenwachstumsfaktors, wobei der native KGF mit der folgenden Sequenz korrespondiert 
Cys Asn Asp Met Thr Pro Glu Gln Met Ala Thr Asn Val Asn Cys Ser Ser
1               5                   10                  15

Pro Glu Arg His Thr Arg Ser Tyr Asp Tyr Met Glu Gly Gly Asp Ile
        20                  25                  30

Arg Val Arg Arg Leu Phe Cys Arg Thr Gln Trp Tyr Leu Arg Ile Asp
    35                  40                  45

Lys Arg Gly Lys Val Lys Gly Thr Gln Glu Met Lys Asn Asn Tyr Asn
50                  55                  60                  65

Ile Met Glu Ile Arg Thr Val Ala Val Gly Ile Val Ala Ile Lys Gly
                70                  75                  80

Val Glu Ser Glu Phe Tyr Leu Ala Met Asn Lys Glu Gly Lys Leu Tyr
            85                  90                  95

Ala Lys Lys Glu Cys Asn Glu Asp Cys Asn Phe Lys Glu Leu Ile Leu
        100                 105                 110

Glu Asn His Tyr Asn Thr Tyr Ala Ser Ala Lys Trp Thr His Asn Gly
    115                 120                 125

Gly Glu Met Phe Val Ala Leu Asn Gln Lys Gly Ile Pro Val Arg Gly
130                 135                 140                 145

Lys Lys Thy Lys Lys Glu Gln Lys Thr Ala His Phe Leu Pro Met Ala
150 155 160

Ile Thr,
 mit oder ohne einer Signalsequenz, wobei das Analogon die Aminosäuresequenz von KGF umfasst, die eine Deletion, Substitution und/oder Insertion von einer oder mehreren der Aminosäuren 1 - 24 umfasst, wobei der Cysteinrest in Aminosäureposition 1 und der Cysteinrest in Aminosäureposition 15 jeweils entweder deletiert oder mit einer anderen Aminosäure substituiert sind, das optional zusätzlich einen N-terminalen Methioninrest aufweist und/oder zusätzlich eine Ladungswechsel-Substitution an einer Aminosäureposition ausgewählt aus Arg (41), Glu (43), Cys (55), Cys (95), Asn (137), Glu (138), Cys (139), Arg (144), Cys (147), Glu (152), Cys (153) oder Thr (154) umfasst, oder im Falle von Δ23-KGF eine Ladungswechselmodifikation von His (116) aufweist, oder im Falle von nativem KGF, bei dem beide, Cys (1) und Cys (15) durch Ser substituiert sind, optional auch Cys (40) oder Cys (102) oder beide, Cys (102) und Cys (106), durch Ser substituiert sind, mit der Proviso, dass das Analogon nicht das Keratinocytenwachstumsfaktorprotein, bestehend aus den Aminosäuren 24-163, ist.

2. Das Analogon gemäß Anspruch 1, wobei der Cysteinrest in Aminosäureposition 1 und der Cysteinrest in Aminosäureposition 15 deletiert sind.

3. Das Analogon gemäß Anspruch 1, wobei die Aminosäuren 1 - 24 deletiert sind.

4. Das Analogon gemäß Anspruch 1, wobei der Cysteinrest in Aminosäureposition 1 deletiert ist und der Cysteinrest in Aminosäureposition 15 mit einer anderen Aminosäure substituiert ist.

5. Das Analogon gemäß Anspruch 1, wobei mindestens eine Aminosäure der Aminosäuren 1 bis 24 deletiert ist und der Cysteinrest in Aminosäureposition 15 mit einer anderen Aminosäure substituiert ist.

6. Das Analogon gemäß Anspruch 1, wobei der Cysteinrest in Aminosäureposition 1 und der Cysteinrest in Aminosäureposition 15 mit einer anderen Aminosäure substituiert sind.

7. Das Analogon gemäß einem der Ansprüche 4 bis 6, wobei ein Aminosäurerest, ausgewählt aus Alanin, Leucin oder Serin für einen Cysteinrest substituiert ist.

8. Das Analogon gemäß Anspruch 7, wobei ein Serinrest für einen Cysteinrest substituiert ist.

9. Das Analogen gemäß einem der Ansprüche 1 bis 8, umfassend eine Ladungswechsel-Substitution an einer Aminosäureposition, ausgewählt aus dem Argininrest in der Aminosäureposition 41, den Glutaminrest in der Aminosäureposition 43, dem Lysinrest in der Aminosäureposition 55, dem Lysinrest an der Aminosäureposition 95, dem Asparaginrest in der Aminosäureposition 137, dem Glutaminrest in der Aminosäureposition 138, dem Lysinrest in der Aminosäureposition 139, dem Argininrest in der Aminosäureposition 144, dem Lysinrest in der Aminosäureposition 147, dem Glutaminrest in der Aminosäureposition 152, dem Lysinrest in der Aminosäureposition 153 oder dem Threoninrest in der Aminosäureposition 154.

10. Das Anatogon gemäß einem der Ansprüche 1 bis 9, umfassend eine Ladungs-Wechsel-Modifikation des Histidinrests in der Aminosäureposition 116.

11. Das Analogon gemäß Anspruch 10, wobei der Histidinrest durch einen Glycinrest ersetzt wird.

12. Das Analogon gemäß einem der Ansprüche 9 bis 11, wobei die Aminosäuren 1-24 deletiert sind.

13. Das Analogon gemäß Anspruch 1, wobei das Analogon C(1,15)S; C(1,15,40)S; C(1,15,102)S; C(1,15,102,106)S; ΔN15; ΔN16; ΔN17; ΔN18; ΔN19; ΔN20; ΔN21; ΔN22; ΔN23; ΔN24; ΔN3/C(15)S; ΔN3(C(15)-; ΔN8/C(15)S; ΔN8/C(15)- und ΔN23/H(116)G ist, welches zu den Aminosäuren 23 - 163 korrespondiert, wobei der Histidinrest in Aminosäureposition 116 mit einem Glycin substituiert ist.

14. Das Analogon gemäß Anspruch 13, wobei besagtes Analogon ΔN16 ist.

15. Das Analogon gemäß einem der Ansprüche 1 bis 14, wobei besagtes Analogon eine Signalsequenz oder einen aminoterminalen Methioninrest aufweist.

16. Das Analogon gemäß Anspruch 15, wobei besagte Signalsequenz die Aminosäuren 1 - 31 von Fig. 1 ist.

17. Das Analogon gemäß einem der Ansprüche 1 - 16, wobei besagtes Analogon kovalent an Polyethylenglycol oder ein verwandtes wasserlösliches organisches Polymer gebunden ist.

18. Das Analogon gemäß Anspruch 17, wobei das Analogon an Polyethylenglycol gebunden ist.

19. Ein Analogon gemäß Anspruch 1, wobei besagtes Analogon ΔN23 ist, das kovalent an Polyethylenglycol oder verwandte wasserlösliche organische Polymere gebunden ist.

20. Das Analogon gemäß Anspruch 19, wobei das Analogon an Polyethylenglycol gebunden ist.

21. Das Analogon gemäß einem der Ansprüche 1 bis 20, wobei besagtes Analogon lyophilisiert ist.

22. Eine pharmazeutische Formulierung, umfassend eine therapeutisch wirksame Menge eines Analogons von KGF gemäß einem der Ansprüche 1 bis 21 und einen pharmazeutisch verträglichen Träger.

23. Ein rekombinantes Nukleinsäuremolekül, das ein Analogon gemäß einem der Ansprüche 1 bis 16 kodiert.

24. Ein biologisch funktioneller Vektor, umfassend ein Nukleinsäuremolekül gemäß Anspruch 23.

25. Eine prokaryontische oder eukaryontische Wirtszelle, enthaltend ein Nukleinsäuremolekül gemäß Anspruch 23 oder einen biologisch funktionellen Vektor gemäß Anspruch 24.

26. Eine prokaryontische Wirtszelle gemäß Anspruch 25, die *E. coli* ist.

27. Eine eukaryontische Wirtszelle gemäß Anspruch 25, die eine Säugetierzelle ist, vorzugsweise eine Chinesische Hamsterovarzelle.

28. Ein Verfahren für die Herstellung eines Analogons von KGF, wobei das Verfahren das Wachsen einer Wirtszelle gemäß einem der Ansprüche 25 bis 27 unter geeigneten Nährstoffbedingungen in einer Weise umfasst, die die Expression des kodierten Analogons erlaubt und das Isolieren des so hergestellten Analogons.

29. Eine Verwendung einer wirksamen Menge des Analogons von KGF gemäß einem der Ansprüche 1 bis 21 zur Herstellung eines Medikaments zur Stimulation der Produktion von Nicht-Fibroblastenepithelzellen.

30. Die Verwendung gemäß Anspruch 29, wobei besagte Nicht-Fibroblastenepithelzellen ausgewählt sind aus adnexalen Strukturen, Leberzellen, Mukosaepithel in den respiratorischen und gastrointestinalen Trakten, Komeazellen oder tympanische Epithelzellen.

31. Eine Verwendung einer wirksamen Menge des Analogons von KGF gemäß einem der Ansprüche 1 bis 21 für die Herstellung eines Medikaments zur Stimulation der Produktion von Nicht-Fibroblastenepithelzellen in einem Patienten für die Verhinderung oder Behandlung eines Zustands, wobei besagter Zustand ausgewählt ist aus Verbrennungen und anderen Verletzungen teilweiser oder vollständiger Dicke; Epidermolyse bullosa; Chemotherapie-induzierte Alopecia; männliche Musterglatzköpfigkeit; progressiver Verlust der Haare in Männern und Frauen; Magenund Zwölffingerdarmgeschwüre; entzündliche Darmkrankheiten wie Crohn's Krankheit und geschwürartiger Darmkatarrh; Darmtoxizität bei Bestrahlungs- und chemotherapeutischen Behandlungen; hyaline Membranerkrankung; akuter oder chronischer Lungenschaden; Leberzirrhose; fulminantes Leberversagen; akute virale Hepatitis; toxische Angriffe auf die Leber; Korneaabrieb; progressive Mundschleimhauterkrankung oder Trommelfellschaden.

32. Ein *In-vitro*-Verfahren zur Stimulierung der Produktion von Nicht-Fibroblastenepithefzellen, umfassend die Verabreichung einer wirksamen Menge eines Analogons von KGF gemäß einem der Ansprüche 1 bis 21 oder einer pharmazeutischen Zusammensetzung von Anspruch 22.

33. Die Verwendung von Anspruch 29, wobei die Nicht-Fibroblastenepithelzellen in einem Patienten, der dessen bedarf, stimuliert werden.

34. Das Verfahren von Anspruch 32, wobei besagte Nicht-Fibroblastenepithelzellen ausgewählt sind aus adnexalen Strukturen, Leberzellen, Mukosaepithel in den respiratorischen und gastrointestinalen Trakten, Komeazellen oder tympanischen Epithelzellen.

35. Verwendung einer wirksamen Menge von ΔN23 für die Herstellung eines Medikaments zur Stimulierung der Produktion von Nicht-Fibroblastenepithelzellen in einem Patienten, der dessen bedarf, wobei die Nicht-Fibroblastenepithelzellen ausgewählt sind aus adnexalen Strukturen, Leberzellen, Mukosaepithel in dem respiratorischen Trakt oder tympanische Epithelzellen.

36. Ein *In-vitro*-Verfahren zur Stimulation der Produktion von Nicht-Fibroblastenepithelzellen, umfassend die Verabreichung einer wirksamen Menge von ΔN23 oder einer pharmazeutischen Zusammensetzung davon, wobei die Nicht-Fibroblastenepithelzellen ausgewählt sind aus adnexalen Strukturen, Leberzellen, Mukosaepithel aus dem Atemwegstrakt oder tympanischen Epithelzellen.

37. Verwendung einer wirksamen Menge von ΔN23 für die Herstellung eines Medikaments zur Stimulation der Produktion von Nicht-Fibroblastenepithelzellen in einem Patienten für die Verhinderung oder Behandlung eines Zustands, wobei besagter Zustand ausgewählt ist aus Verbrennungen und anderen Verletzungen teilweiser oder vollständiger Dicke; Epidermolyse bullosa; Chemotherapie-induzierte Alopecia; männliche Musterglatzköpfigkeit; progressiver Verlust der Haare in Männern und Frauen; Magen- und Zwölffingerdarmgeschwüre; entzündliche Darmkrankheiten wie Crohn's Krankheit und geschwürartiger Darmkatarrh; Darmtoxizität bei Bestrahlungs- und chemotherapeutischen Behandlungen; hyaline Membranerkrankung; akuter oder chronischer Lungenschaden; Leberzirrhose; fulminantes Leberversagen; akute virale Hepatitis; toxische Angriffe auf die Leber; Korneaabrieb; progressive Mundschleimhauterkrankung oder Trommelfellschaden.

38. Die Verwendung gemäß einem der Ansprüche 29, 30, 31, 33, 35 und 37, wobei besagtes Analogon von KGF mittels IV, IT, IM, SC oder IP verabreicht wird.

39. Ein Kit, umfassend ein Analogon von KGF gemäß einem der Ansprüche 1 bis 21 oder eine pharmazeutische Formulierung von Anspruch 22.

## Revendications

1. Analogue polypeptidique du facteur de croissance kératocytaire ("KGF") natif, dans lequel le KGF natif correspond à la séquence suivante
avec ou sans séquence signal, dans lequel l'analogue comporte la séquence peptidique du KGF comportant une délétion, une substitution et/ou une insertion d'un ou plusieurs acides aminés 1-24, où le résidu cystéine en position 1 et le résidu cystéine en position 15 sont, chacun, délété ou remplacé par un autre acide aminé, ayant en outre, facultativement, un résidu méthionine N-terminal et/ou comportant en outre une substitution changeant la charge en une position dans la chaîne peptidique sélectionnée parmi Arg(41), Glu(43), Lys(55), Lys(95), Asn(137), Glu(138), Lys(139), Arg(144), Lys(147), Gln(152), Lys(153) ou Thr(154) ou, en cas de Δ23-KGF, ayant une modification changeant la charge de His(116) ou, au cas où le KGF natif aurait à la fois une Cys(1) et une Cys(15) remplacée par une Ser, ayant également la Cys(40) ou la Cys(102) ou à la fois la Cys(102) et la Cys(106) remplacées par une Ser, avec cette réserve que l'analogue n'est pas le facteur de croissance kératinocytaire composé des acides aminés 24-163.

2. Analogue selon la revendication 1, dans lequel le résidu cystéine en position 1 et le résidu cystéine en position 15 sont délétés.

3. Analogue selon la revendication 1, dans lequel les acides aminés 1 à 24 sont délétés.

4. Analogue selon la revendication 1, dans lequel le résidu cystéine en position 1 est délété et le résidu cystéine en position 15 est remplacé par un autre acide aminé.

5. Analogue selon la revendication 1, dans lequel au moins un acide aminé des acides aminés 1 à 24 est délété et le résidu cystéine en position 15 est remplacé par un autre acide aminé.

6. Analogue selon la revendication 1, dans lequel le résidu cystéine en position 1 et le résidu cystéine en position 15 sont remplacés par un autre acide aminé.

7. Analogue selon l'une quelconque des revendications 4 à 6, dans lequel un résidu acide aminé sélectionné parmi l'alanine, la leucine ou la sérine remplace un résidu cystéine.

8. Analogue selon la revendication 7, dans lequel un résidu serine remplace un résidu cystéine.

9. Analogue selon l'une quelconque des revendications 1 à 8, comportant une substitution changeant la charge en une position sélectionnée à partir du résidu arginine en position 41, du résidu glutamine en position 43, du résidu lysine en position 55, du résidu lysine en position 95, du résidu asparagine en position 137, du résidu glutamine en position 138, du résidu lysine en position 139, du résidu arginine en position 144, du résidu lysine en position 147, du résidu glutamine en position 152, du résidu lysine en position 153 ou du résidu thréonine en position 154.

10. Analogue selon l'une quelconque des revendications 1 à 9, comportant une modification changeant la charge du résidu histidine en position 116.

11. Analogue selon la revendication 10, dans lequel le résidu histidine est remplacé par un résidu glycine.

12. Analogue selon l'une quelconque des revendications 9 à 11, dans lequel les acides aminés 1 à 24 sont délétés.

13. Analogue selon la revendication 1, dans lequel l'analogue est C(1,15)S ; C(1,15,40)S ; C(1,15,102)S ; C(1,15,102,106)S ; ΔN15 ; ΔN16 ; ΔN17 ; ΔN18 ; ΔN19 ; ΔN20 ; ΔN21 ; ΔN22 ; ΔN24 ; ΔN3/C(15)S ; ΔN3/C(15)- ; ΔN8/C(15)S ; ΔN8/C(15)- et ΔN23/H(116)G, ce qui correspond aux acides aminés 23-163, avec le résidu histidine en position 116 substitué par une glycine.

14. Analogue selon la revendication 13, dans lequel ledit analogue est ΔN16.

15. Analogue selon l'une quelconque des revendications 1 à 14, dans lequel ledit analogue a une séquence signal ou un résidu méthionine en position N-terminale.

16. Analogue selon la revendication 15, dans lequel ladite séquence signal est la séquence des acides aminés 1 à 31 de la figure 1.

17. Analogue selon l'une quelconque des revendications 1 à 16, dans lequel ledit analogue est lié de façon covalente au polyéthylène glycol ou à des polymères organiques hydrosolubles apparentés.

18. Analogue selon la revendication 17, dans lequel l'analogue est lié au polyéthylène glycol.

19. Analogue selon la revendication 1, dans lequel ledit analogue est ΔN23 lié de façon covalente au polyéthylène glycol ou à des polymères organiques hydrosolubles apparentés.

20. Analogue selon la revendication 19, dans lequel l'analogue est relié au polyéthylène glycol.

21. Analogue selon l'une quelconque des revendications 1 à 20, dans lequel ledit analogue est lyophilisé.

22. Formulation pharmaceutique comportant une quantité thérapeutique d'un analogue du KGF selon l'une quelconque des revendications 1 à 21 et un transporteur pharmaceutiquement acceptable.

23. Molécule d'acide nucléique recombinée codant pour un analogue selon l'une quelconque des revendications 1 à 16.

24. Vecteur biologiquement fonctionnel, comportant une molécule d'acide nucléique selon la revendication 23.

25. Cellule-hôte procaryote ou eucaryote contenant une molécule d'acide nucléique selon la revendication 23 ou un vecteur biologiquement fonctionnel selon la revendication 24.

26. Cellule-hôte procaryote selon la revendication 25 qui est *E coli*.

27. Cellule-hôte eucaryote selon la revendication 25 qui est une cellule de mammifère, de préférence une cellule d'ovaire de hamster chinois.

28. Procédé pour la production d'un analogue du KGF, le procédé consistant à cultiver, dans des conditions de nutriments appropriées, une cellule-hôte selon l'une quelconque des revendications 25 à 27 d'une manière permettant l'expression de l'analogue codé et à isoler l'analogue ainsi produit.

29. Utilisation d'une quantité efficace de l'analogue du KGF selon l'une quelconque des revendications 1 à 21 pour la production d'un médicament pour stimuler la production de cellules épithéliales non fibroblastiques.

30. Utilisation selon la revendication 29, dans lequel lesdites cellules épithéliales non fibroblastiques sont sélectionnés à partir de structures annexielles, d'hépatocytes, d'épithélium muqueux des tractus respiratoire et digestif, des cellules de la cornée ou du tympan.

31. Utilisation d'une quantité efficace de l'analogue du KGF selon l'une quelconque des revendications 1 à 21 pour la production d'un médicament pour stimuler la production de cellules épithéliales non fibroblastiques chez un patient pour la prévention ou le traitement d'une affection, ladite affection entrant parmi les patrologies suivantes : brûlures et autres lésions cutanées superficielles ou profondes ; épidermolyse bulleuse ; alopécie induite par la chimiothérapie ; calvitie androgénique ; chute progressive des cheveux chez les deux sexes ; ulcères gastriques et duodénaux ; colopathies inflammatoires de type maladie de Crohn et rectocolite hémorragique ; toxicité intestinale de la radiothérapie et de la chimiothérapie ; maladie des membranes hyalines ; pneumopathies aiguës ou chroniques ; cirrhose hépatique ; hépatite fulminante ; hépatite virale aiguë ; hépatites toxiques ; abrasion cornéenne ; gingivite évolutive ou lésions du tympan.

32. Méthode in vitro de stimulation de la production de cellules épithéliales non fibroblastiques comportant l'administration d'une quantité efficace d'un analogue du KGF, selon l'une quelconque des revendications 1 à 21, ou d'une formulation pharmaceutique selon la revendication 22.

33. Utilisation de la revendication 29, dans lequel les cellules épithéliales non fibroblastiques sont stimulées chez un patient qui le nécessite.

34. Méthode de la revendication 32, dans laquelle lesdites cellules épithéliales non fibroblastiques sont sélectionnées à partir de structures annexielles, d'hépatocytes, d'épithélium muqueux des tractus respiratoire et digestif, de cellules de la cornée ou du tympan.

35. Utilisation d'une quantité efficace de ΔN23 pour la production d'un médicament pour stimuler la production de cellules épithéliales non fibroblastiques chez un patient qui le nécessite, dans lequel les cellules épithéliales non fibroblastiques sont sélectionnées à partir de structures annexiellcs, d'hépatocytes, d'épithélium muqueux des tractus respiratoire et digestif, de cellules de la cornée ou du tympan.

36. Méthode in vitro de stimulation de la production de cellules épithéliales non fibroblastiques comportant l'administration d'une quantité efficace de ΔN23 ou d'une composition pharmaceutique selon la revendication 22, dans laquelle les cellules épithéliales non fibroblastiques sont sélectionnées à partir de structures annexielles, d'hépatocytes, d'épithélium muqueux des tractus respiratoire et digestif, de cellules de la cornée ou du tympan.

37. Utilisation d'une quantité efficace de ΔN23 pour la production d'un médicament pour stimuler la production de cellules épithéliales non fibroblastiques chez un patient pour la prévention ou le traitement d'une affection, ladite affection entrant parmi les pathologies suivantes : brûlures et autres lésions cutanées superficielles ou profondes ; épidermolyse bulleuse ; alopécie induite par la chimiothérapie ; calvitie androgénique ; chute progressive des cheveux chez les deux sexes ; ulcères gastriques et duodénaux ; colopathies inflammatoires de type maladie de Crohn et rectocolite hémorragique ; toxicité intestinale de la radiothérapie et de la chimiothérapie ; maladie des membranes hyalines ; pneumopathies aiguës ou chroniques ; cirrhose hépatique ; hépatite fulminante ; hépatite virale aiguë ; hépatites toxiques ; abrasion cornéenne ; gingivite évolutive ou lésions du tympan.

38. Utilisation selon l'une quelconque des revendications 29, 30, 31, 33, 35 et 37, dans lequel ledit analogue du KGF est administré par voie IV, IT, IM, SC ou IP.

39. Trousse comportant un analogue du KGF selon l'une quelconque des revendications 1 à 21, ou une formulation pharmaceutique selon la revendication 22.
